# EUROPEAN PATENT APPLICATION

(11) **EP 4 797 143 A1**
(43) Date of publication of application: **26.08.2026**
(21) Application number: 24884090.2
(22) Date of filing: 06.08.2024
(51) Int. Cl.: G06F 30/20, C21B 5/00, G01D 21/02, G01K 13/00, G16C 20/30, G16C 20/10

(54) **METHOD AND APPARATUS FOR PREDICTING TEMPERATURE OF MOLTEN IRON IN BLAST FURNACE SMELTING, ELECTRONIC DEVICE, AND STORAGE MEDIUM**

(30) Priority: 01.11.2023 CN 202311447213
(71) Applicant: CISDI Engineering Co., Ltd, Chongqing 400013 (CN)
(72) Inventor: HE, Kun, Chongqing 400013 (CN); WANG, Gang, Chongqing 400013 (CN); ZOU, Zhongping, Chongqing 400013 (CN); ZHAO, Yunjian, Chongqing 400013 (CN); NIU, Qun, Chongqing 400013 (CN); QUAN, Kui, Chongqing 400013 (CN)
(74) Representative: Chung, Hoi Kan
(86) International application number: PCT/CN2024/110031
(87) International publication number: WO 2025/092083

(57) **Abstract**

The present invention provides a method, an apparatus, an electronic device, and a storage medium for predicting molten iron temperature in a blast furnace smelting process. The method comprises acquiring raw fuel parameters, blast parameters, gas injection parameters, top gas parameters, and slag-metal parameters during the blast furnace smelting process; determining, based on the raw fuel parameters, the blast parameters, and the gas injection parameters, a belly gas flow rate, a belly gas composition, and a theoretical combustion temperature of gas in a tuyere raceway zone; determining, based on the top gas parameters, the slag-metal parameters, the belly gas flow rate, and the belly gas composition, a direct reduction degree of iron ore; determining, based on the theoretical combustion temperature of gas, the belly gas composition, the slag-metal parameters, and a post-direct-reduction gas composition, a gas-liquid heat exchange coefficient; and performing iterative solving of molten iron temperature and gas temperature based on the gas-liquid heat exchange coefficient, the direct reduction degree of iron ore, the theoretical combustion temperature of gas, and the slag-metal parameters, so as to complete prediction of the molten iron temperature in blast furnace smelting. The method improves the accuracy of molten iron temperature prediction, provides a reliable basis for blast furnace smelting control, and ensures stable and smooth operation of the blast furnace.

## Description

### TECHNICAL FIELD

The present application relates to the technical field of blast furnace smelting, and in particular relates to a blast furnace smelting molten iron temperature prediction method, apparatus, electronic device, and storage medium.

### BACKGROUND OF THE INVENTION

In a blast furnace smelting process, the molten iron temperature can reflect a thermal balance state inside a blast furnace and a reduction state of iron ore. Meanwhile, gas injection technology is a key technology for achieving carbon reduction in blast furnace smelting, and maintaining a stable molten iron temperature is an important condition for stable and smooth operation of a blast furnace under a gas injection operating condition. Therefore, effectively and accurately predicting the molten iron temperature has important guiding significance for operation control of blast furnace smelting.

At present, related technologies mostly determine a variation pattern of the molten iron temperature based on historical operation data, and then predict the molten iron temperature. However, consideration of real-time parameters is insufficient, which affects prediction accuracy and makes it difficult to reasonably predict the molten iron temperature under a gas injection operating condition.

### SUMMARY OF THE INVENTION

In view of the above-described deficiencies of the prior art, the present application provides a blast furnace smelting molten iron temperature prediction method, apparatus, electronic device, and storage medium, so as to solve the technical problem that, when predicting molten iron temperature, related technologies give insufficient consideration to real-time parameters, which affects prediction accuracy and makes it difficult to reasonably predict the molten iron temperature under a gas injection operating condition.

The present application provides a blast furnace smelting molten iron temperature prediction method. The method comprises: obtaining a raw fuel parameter, a blast parameter, a gas injection parameter, a top gas parameter, and a slag-iron parameter in a blast furnace smelting process; determining, according to the raw fuel parameter, the blast parameter, and the gas injection parameter, total heat input, bosh gas volume, and bosh gas composition of a tuyere raceway, so as to determine theoretical combustion temperature of gas in the tuyere raceway according to the total heat input, the bosh gas volume, and the bosh gas composition; determining a direct reduction degree of iron ore according to the top gas parameter, the slag-iron parameter, the bosh gas volume, and the bosh gas composition; determining a gas-liquid heat exchange coefficient according to the theoretical combustion temperature of gas, the bosh gas composition, the slag-iron parameter, and gas composition after direct reduction at a direct reduction position, wherein the gas composition after direct reduction is determined based on the top gas parameter, or the gas composition after direct reduction is determined based on the top gas parameter, the bosh gas volume, and the bosh gas composition; and performing iterative solution on the molten iron temperature and the gas temperature based on the gas-liquid heat exchange coefficient, the direct reduction degree of iron ore, the theoretical combustion temperature of gas, and the slag-iron parameter until a first convergence condition is satisfied, so as to complete blast furnace smelting molten iron temperature prediction.

In an embodiment of the present application, determining, according to the raw fuel parameter, the blast parameter, and the gas injection parameter, the total heat input, the bosh gas volume, and the bosh gas composition of the tuyere raceway, so as to determine the theoretical combustion temperature of gas in the tuyere raceway according to the total heat input, the bosh gas volume, and the bosh gas composition comprises: determining, according to the raw fuel parameter, the blast parameter, and the gas injection parameter, a volume of gas participating in a chemical reaction, a volume of gas not participating in the chemical reaction, and a volume of gas generated by the chemical reaction in the tuyere raceway; calculating heat released by the chemical reaction in the tuyere raceway based on the volume of gas participating in the chemical reaction, and calculating the total heat input according to coke sensible heat, blast sensible heat, gas sensible heat, and the heat released by the chemical reaction, wherein the coke sensible heat is determined based on the raw fuel parameter, the blast sensible heat is determined based on the blast parameter, and the gas sensible heat is determined based on the gas injection parameter; calculating the bosh gas volume and the bosh gas composition according to the volume of gas generated by the chemical reaction and the volume of gas not participating in the chemical reaction; and performing iterative solution on the theoretical combustion temperature of gas based on the bosh gas volume, the bosh gas composition, and the total heat input until a second convergence condition is satisfied, so as to obtain the theoretical combustion temperature of gas.

In an embodiment of the present application, before calculating the total heat input according to the coke sensible heat, the blast sensible heat, the gas sensible heat, and the heat released by the chemical reaction in the tuyere raceway, the method further comprises: determining a coke specific heat capacity according to a preset coke temperature and coke composition, so as to calculate the coke sensible heat according to the preset coke temperature, the coke specific heat capacity, and a coke amount, wherein the raw fuel parameter comprises the coke composition and the coke amount; determining a blast specific heat capacity according to a blast temperature and blast composition, so as to calculate the blast sensible heat according to the blast specific heat capacity, a blast amount, and the blast temperature, wherein the blast parameter comprises the blast composition, the blast temperature, and the blast amount; and determining a gas injection specific heat capacity according to a preset gas injection temperature and gas injection composition, so as to calculate the gas sensible heat according to the preset gas injection temperature, the gas injection specific heat capacity, and a gas injection amount, wherein the gas injection parameter comprises the gas injection composition and the gas injection amount.

In an embodiment of the present application, performing iterative solution on the theoretical combustion temperature of gas based on the bosh gas volume, the bosh gas composition, and the total heat input until the second convergence condition is satisfied, so as to obtain the theoretical combustion temperature of gas, comprises: determining an initial bosh gas specific heat capacity of the tuyere raceway according to the bosh gas composition and a preset theoretical combustion temperature of gas, and calculating an initial theoretical combustion temperature of gas according to the total heat input, the bosh gas volume, and the initial bosh gas specific heat capacity; using the initial theoretical combustion temperature of gas as a pre-iteration theoretical combustion temperature of gas, determining an iterative bosh gas specific heat capacity of the tuyere raceway according to the bosh gas composition and the pre-iteration theoretical combustion temperature of gas, and calculating a post-iteration theoretical combustion temperature of gas according to the total heat input, the bosh gas volume, and the iterative bosh gas specific heat capacity; calculating a difference between the pre-iteration theoretical combustion temperature of gas and the post-iteration theoretical combustion temperature of gas to obtain an iteration temperature difference of the theoretical combustion temperature of gas; if the iteration temperature difference of the theoretical combustion temperature of gas is less than or equal to a second preset temperature difference threshold, determining that the second convergence condition is satisfied, and using the post-iteration theoretical combustion temperature of gas as the theoretical combustion temperature of gas; and if the iteration temperature difference of the theoretical combustion temperature of gas is greater than the second preset temperature difference threshold, performing iterative solution on the theoretical combustion temperature of gas based on the bosh gas volume, the bosh gas composition, the total heat input, and the post-iteration theoretical combustion temperature of gas until the second convergence condition is satisfied, so as to obtain the theoretical combustion temperature of gas.

In an embodiment of the present application, determining the direct reduction degree of iron ore according to the top gas parameter, the slag-iron parameter, the bosh gas volume, and the bosh gas composition comprises: determining an amount of CO in bosh gas based on the bosh gas volume and the bosh gas composition, and determining a total amount of CO and CO₂ in top gas based on a top gas volume and a top gas composition, so as to determine a total amount of CO generated by direct reduction according to the amount of CO in bosh gas and the total amount of CO and CO₂ in top gas, wherein the top gas parameter comprises the top gas volume and the top gas composition; determining a mass of trace elements in molten iron and a mass of iron in molten iron based on a molten iron composition and a molten iron yield, and determining an amount of CO generated by direct reduction of trace element oxides based on the mass of trace elements in molten iron, so as to determine an amount of CO generated by direct reduction of iron ore according to the total amount of CO generated by direct reduction and the amount of CO generated by direct reduction of trace element oxides, wherein the slag-iron parameter comprises the molten iron composition and the molten iron yield; and calculating the direct reduction degree of iron ore based on the amount of CO generated by direct reduction of iron ore, the mass of iron in molten iron, and a mass of iron in a preset hot briquetted iron block.

In an embodiment of the present application, before determining the gas-liquid heat exchange coefficient, the method comprises: determining a total amount of CO and CO₂ in top gas, a total amount of H₂O and H₂ in top gas, and an amount of N₂ in top gas based on a top gas volume and a top gas composition, wherein the top gas parameter comprises the top gas volume and the top gas composition; using the top gas volume as a gas volume after direct reduction, using the total amount of CO and CO₂ in top gas as an amount of CO in gas after direct reduction, using the total amount of H₂O and H₂ in top gas as an amount of H₂ in gas after direct reduction, and using the amount of N₂ in top gas as an amount of N₂ in gas after direct reduction; and determining the gas composition after direct reduction based on the gas volume after direct reduction, the amount of CO in gas after direct reduction, the amount of H₂ in gas after direct reduction, and the amount of N₂ in gas after direct reduction.

In an embodiment of the present application, before determining the gas-liquid heat exchange coefficient, the method comprises: determining a total amount of CO and CO₂ in top gas based on a top gas volume and a top gas composition, wherein the top gas parameter comprises the top gas volume and the top gas composition; determining an amount of H₂ in bosh gas and an amount of N₂ in bosh gas based on the bosh gas volume and the bosh gas composition; using the top gas volume as a gas volume after direct reduction, using the total amount of CO and CO₂ in top gas as an amount of CO in gas after direct reduction, using the amount of H₂ in bosh gas as an amount of H₂ in gas after direct reduction, and using the amount of N₂ in bosh gas as an amount of N₂ in gas after direct reduction; and determining the gas composition after direct reduction based on the gas volume after direct reduction, the amount of CO in gas after direct reduction, the amount of H₂ in gas after direct reduction, and the amount of N₂ in gas after direct reduction.

In an embodiment of the present application, determining the gas-liquid heat exchange coefficient according to the theoretical combustion temperature of gas, the bosh gas composition, the slag-iron parameter, and the gas composition after direct reduction at a direct reduction position comprises: performing interpolation calculation on the bosh gas composition and the gas composition after direct reduction to obtain a gas composition of a dripping zone; determining a gas-liquid heat exchange coefficient of the tuyere raceway according to a slag-iron equivalent particle size, a thermal conductivity coefficient of gas in the tuyere raceway, and a Nusselt number of the tuyere raceway, wherein the thermal conductivity coefficient of gas in the tuyere raceway and the Nusselt number of the tuyere raceway are both determined based on the bosh gas composition and the theoretical combustion temperature of gas, and the slag-iron equivalent particle size is determined based on the slag-iron parameter; determining a gas-liquid heat exchange coefficient at the direct reduction position according to the slag-iron equivalent particle size, a thermal conductivity coefficient of gas at the direct reduction position, and a Nusselt number at the direct reduction position, wherein the thermal conductivity coefficient of gas at the direct reduction position and the Nusselt number at the direct reduction position are both determined based on the gas composition after direct reduction and the theoretical combustion temperature of gas; and determining a gas-liquid heat exchange coefficient of the dripping zone according to the slag-iron equivalent particle size, a thermal conductivity coefficient of gas in the dripping zone, and a Nusselt number of the dripping zone, wherein the thermal conductivity coefficient of gas in the dripping zone and the Nusselt number of the dripping zone are both determined based on the gas composition of the dripping zone and the theoretical combustion temperature of gas.

In an embodiment of the present application, performing iterative solution on the molten iron temperature and the gas temperature based on the gas-liquid heat exchange coefficient, the direct reduction degree of iron ore, the theoretical combustion temperature of gas, and the slag-iron parameter until the first convergence condition is satisfied, so as to complete molten iron temperature prediction, comprises: determining direct reduction heat consumption according to a molten iron composition and the direct reduction degree of iron ore, wherein the slag-iron parameter comprises the molten iron composition; performing iterative solution on the molten iron temperature and the gas temperature based on a gas phase temperature, a molten iron phase temperature, the gas-liquid heat exchange coefficient, and the direct reduction heat consumption, and according to a gas phase energy conservation relationship and a molten iron phase energy conservation relationship, to obtain a pre-iteration molten iron temperature of the tuyere raceway, a post-iteration molten iron temperature of the tuyere raceway, a pre-iteration gas temperature after direct reduction, and a post-iteration gas temperature after direct reduction, wherein an initial value of the gas phase temperature is the theoretical combustion temperature of gas, and an initial value of the molten iron phase temperature is a preset molten iron temperature after direct reduction; determining a difference between the pre-iteration molten iron temperature of the tuyere raceway and the post-iteration molten iron temperature of the tuyere raceway as a molten iron temperature iteration difference, and determining a difference between the pre-iteration gas temperature after direct reduction and the post-iteration gas temperature after direct reduction as a gas temperature iteration difference; and if the molten iron temperature iteration difference and the gas temperature iteration difference are both less than or equal to a first preset temperature difference threshold, determining that the first convergence condition is satisfied, and using the post-iteration molten iron temperature of the tuyere raceway as a final prediction result of the molten iron temperature prediction.

In an embodiment of the present application, a blast furnace smelting molten iron temperature prediction apparatus is further provided. The apparatus comprises: a smelting data acquisition module, configured to obtain a raw fuel parameter, a blast parameter, a gas injection parameter, a top gas parameter, and a slag-iron parameter in a blast furnace smelting process; a theoretical combustion temperature determination module, configured to determine, according to the raw fuel parameter, the blast parameter, and the gas injection parameter, total heat input, bosh gas volume, and bosh gas composition of a tuyere raceway, so as to determine theoretical combustion temperature of gas in the tuyere raceway according to the total heat input, the bosh gas volume, and the bosh gas composition; a direct reduction degree determination module, configured to determine a direct reduction degree of iron ore according to the top gas parameter, the slag-iron parameter, the bosh gas volume, and the bosh gas composition; a heat exchange coefficient determination module, configured to determine a gas-liquid heat exchange coefficient according to the theoretical combustion temperature of gas, the bosh gas composition, the slag-iron parameter, and gas composition after direct reduction at a direct reduction position, wherein the gas composition after direct reduction is determined based on the top gas parameter, or is determined based on the top gas parameter, the bosh gas volume, and the bosh gas composition; and an iterative solution module, configured to perform iterative solution on the molten iron temperature and the gas temperature based on the gas-liquid heat exchange coefficient, the direct reduction degree of iron ore, the theoretical combustion temperature of gas, and the slag-iron parameter until the first convergence condition is satisfied, so as to complete blast furnace smelting molten iron temperature prediction.

In an embodiment of the present application, an electronic device is further provided. The electronic device comprises one or more processors and a storage device configured to store one or more programs. When the one or more programs are executed by the one or more processors, the electronic device is enabled to implement the above-described blast furnace smelting molten iron temperature prediction method.

In an embodiment of the present application, a computer-readable storage medium is further provided. The computer-readable storage medium stores a computer program, and when the computer program is executed by a processor of a computer, the computer is caused to execute the above-described blast furnace smelting molten iron temperature prediction method.

### Beneficial Effects

The present invention provides a blast furnace smelting molten iron temperature prediction method, apparatus, electronic device, and storage medium. The method performs calculation based on raw fuel parameters, blast parameters, gas injection parameters, top gas parameters, and slag-iron parameters in a blast furnace smelting process, so as to obtain a theoretical combustion temperature of gas, a gas-liquid heat exchange coefficient, and a direct reduction degree of iron ore, and further performs iterative solution on molten iron temperature and gas temperature. As a result, molten iron temperature prediction under various operating conditions, including gas injection operating conditions, can be achieved, and prediction accuracy of the molten iron temperature can be improved, thereby providing a reliable basis for operation control of blast furnace smelting and ensuring stable and smooth operation of the blast furnace.

It should be understood that the above general description and the following detailed description are merely exemplary and explanatory, and are not intended to limit the present application.

### BRIEF DESCRIPTION OF DRAWINGS

FIG. 1 is a flowchart of a blast furnace smelting molten iron temperature prediction method according to an exemplary embodiment of the present application.
FIG. 2 is a schematic structural diagram of a blast furnace smelting molten iron temperature prediction system according to a specific embodiment of the present application.
FIG. 3 is a schematic diagram of a blast furnace smelting molten iron temperature prediction process of the blast furnace smelting molten iron temperature prediction system shown in FIG. 2 according to a specific embodiment.
FIG. 4 is a block diagram of a blast furnace smelting molten iron temperature prediction apparatus according to an exemplary embodiment of the present application.
FIG. 5 is a schematic structural diagram of an electronic device according to an exemplary embodiment of the present application.

### DETAILED DESCRIPTION OF THE INVENTION

The embodiments of the present application are described below through specific examples. Those skilled in the art can readily understand other advantages and effects of the present application from the contents disclosed in this specification. The present application may also be implemented or applied through other different specific embodiments, and various details described in this specification may be modified or changed based on different viewpoints and applications without departing from the spirit of the present application. It should be noted that, unless otherwise conflicting, the embodiments described below and features in the embodiments may be combined with one another.

It should be noted that the drawings provided in the following embodiments are merely schematic illustrations of the basic concepts of the present application. Accordingly, the drawings only show components related to the present application and are not drawn according to the actual number, shape, or size of components in practical implementation. In actual implementation, the form, quantity, and proportion of each component may be arbitrarily changed, and the layout of the components may be more complex.

It should further be noted that terms such as "first" and "second" in the present application are merely used to distinguish similar objects and do not indicate any particular order or sequence. Terms such as "including" and "having" and variations thereof are intended to indicate that the subject matter encompasses the listed elements and equivalents thereof, without excluding other elements.

It can be understood that various numerical identifiers, step numbers, and reference numerals described in the present application are used merely for convenience of description and are not intended to limit the scope of the present application. The magnitude of the reference numerals does not imply an execution order, and the execution order of each process should be determined based on its function and internal logic.

In the following description, numerous specific details are set forth to provide a thorough understanding of the embodiments of the present application. However, it will be apparent to those skilled in the art that the embodiments of the present application may be practiced without these specific details. In other embodiments, well-known structures and devices are shown in the form of block diagrams rather than detailed descriptions in order to avoid obscuring the embodiments of the present application.

The embodiments of the present application respectively provide a method for predicting molten iron temperature in blast furnace smelting, a blast furnace smelting molten iron temperature prediction apparatus, an electronic device, a computer-readable storage medium, and a computer program product. These embodiments will be described in detail below.

In one embodiment of the present application, a method for predicting molten iron temperature in blast furnace smelting is provided, comprising: obtaining a raw fuel parameter, a blast parameter, a gas injection parameter, a top gas parameter, and a slag-iron parameter in a blast furnace smelting process; determining, according to the raw fuel parameter, the blast parameter, and the gas injection parameter, total heat input, bosh gas volume, and bosh gas composition of a tuyere raceway, so as to determine the theoretical combustion temperature of gas in the tuyere raceway based on the total heat input, the bosh gas volume, and the bosh gas composition; determining a direct reduction degree of iron ore according to the top gas parameter, the slag-iron parameter, the bosh gas volume, and the bosh gas composition; determining a gas-liquid heat exchange coefficient according to the theoretical combustion temperature of gas, the bosh gas composition, the slag-iron parameter, and the gas composition after direct reduction at a direct reduction position, wherein the gas composition after direct reduction is determined based on the top gas parameter, or the gas composition after direct reduction is determined based on the top gas parameter, the bosh gas volume, and the bosh gas composition; performing iterative solution on the molten iron temperature and the gas temperature based on the gas-liquid heat exchange coefficient, the direct reduction degree of iron ore, the theoretical combustion temperature of gas, and the slag-iron parameter until the first convergence condition is satisfied, so as to complete the molten iron temperature prediction in blast furnace smelting.

As can be seen, the technical solution of the embodiment of the present application enables molten iron temperature prediction under various operating conditions, such as gas injection operating conditions, and improves the accuracy of molten iron temperature prediction, providing a reliable basis for the operation control of blast furnace smelting to ensure stable and smooth operation of the blast furnace.

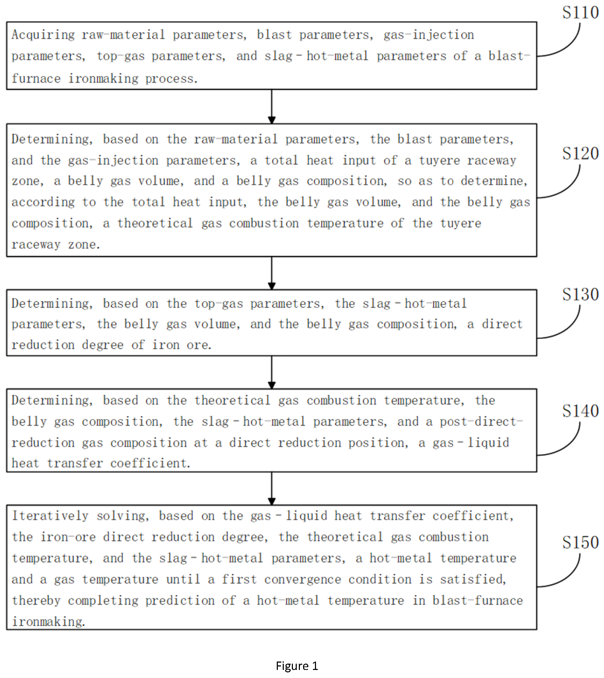

Referring to FIG. 1, FIG. 1 is a flowchart illustrating a method for predicting a molten iron temperature in blast furnace smelting according to an exemplary embodiment of the present application. As shown in FIG. 1, in an exemplary embodiment, the blast furnace molten iron temperature prediction method at least includes steps S110 through S150, which are described in detail below:

Step S110: acquiring raw and fuel material parameters, blast parameters, gas injection parameters, top gas parameters, and slag-iron parameters in a blast furnace smelting process.

In one embodiment of the present application, the raw and fuel material parameters refer to raw and fuel material compositions and raw and fuel material consumption, and may include at least one of coke composition and coke consumption and pulverized coal composition and pulverized coal consumption.wherein the raw and fuel material composition refers to the proportion of constituent substances in the raw and fuel materials. For example, the coke composition may include proportions of C (carbon), H (hydrogen), O (oxygen), N (nitrogen), S (sulfur), volatile matter, and ash in the coke. The pulverized coal composition may include proportions of C, H, O, N, S, volatile matter, and ash in the pulverized coal.

The blast parameters may include at least one of blast volume, blast temperature, and blast composition, wherein the blast composition may include at least one of blast humidity and oxygen enrichment ratio.

The gas injection parameters may include at least one of type of injected medium, injected gas composition, injected gas flow rate, and injected gas temperature. The injected medium may include one or a combination of coke oven gas, basic oxygen furnace gas, decarbonized blast furnace gas, natural gas, pure hydrogen, chemical tail gas, and shale gas. The injected gas composition refers to the proportion of constituent substances in the injected gas. For example, the injected gas composition may include proportions of H₂ (hydrogen), CO (carbon monoxide), CO₂ (carbon dioxide), CH₄ (methane), N₂ (nitrogen), and O₂ (oxygen) in the injected gas.

The top gas parameters may include at least one of top gas composition and top gas flow rate, wherein the top gas composition refers to the proportion of constituent substances in the top gas. For example, the top gas composition may include proportions of H₂, CO, CO₂, and N₂ in the top gas.

The slag-iron parameters may include at least one of molten iron composition (i.e., slag-iron composition), molten iron yield, and slag generation amount (i.e., slag amount). The molten iron composition refers to the proportion of constituent substances in the molten iron. For example, the molten iron composition may include proportions of Fe (iron), C (carbon), Si (silicon), Mn (manganese), P (phosphorus), S (sulfur), and Ti (titanium) in the molten iron.

It should be understood that the raw fuel parameters, the blast parameters, the gas injection parameters, the top gas parameters, and the slag-iron parameters are real-time data of the blast furnace smelting process, which can directly and instantaneously reflect the smelting conditions inside the blast furnace. Accordingly, the molten iron temperature can be accurately predicted based on the raw fuel parameters, the blast parameters, the gas injection parameters, the top gas parameters, and the slag-iron parameters.

Step S120: determining, based on the raw fuel parameters, the blast parameters, and the gas injection parameters, a total heat input, a bosh gas volume, and a bosh gas composition of a tuyere raceway zone, so as to determine a theoretical combustion temperature of gas in the tuyere raceway zone according to the total heat input, the bosh gas volume, and the bosh gas composition.

In one embodiment of the present application, the blast furnace includes five regions, sequentially arranged from top to bottom, namely a lumpy zone, a cohesive zone, a dripping zone, a tuyere raceway zone, and a slag-iron zone, which are distributed at different height positions inside the blast furnace. In the lumpy zone, burden moisture evaporation, thermal decomposition, iron ore reduction, and heat exchange between the burden and gas are performed. In the cohesive zone, the burden softens, and molten dripping begins at a lower boundary of the cohesive zone, where direct reduction reactions for blast furnace ironmaking occur to form primary slag; therefore, the lower boundary of the cohesive zone corresponds to a direct reduction position. In the dripping zone, multiple chemical reactions occur among dripping molten slag-iron, gas, and solid carbon. In the tuyere raceway zone, injected fuels combust with hot blast to generate high-temperature gas. In the slag-iron zone, slag-metal reactions occur at interfaces between slag and iron layers and when iron droplets pass through the slag layer.

The slag-iron in the tuyere raceway zone is in a molten state, and the molten slag-iron corresponds to molten iron. Accordingly, the molten iron temperature refers to the temperature of the molten slag-iron in the tuyere raceway zone. Since heat exchange occurs between the molten iron and the gas, the molten iron temperature is affected by the theoretical combustion temperature of gas in the tuyere raceway zone. Based on this, the theoretical combustion temperature of gas needs to be determined. Specifically, the total heat input, the bosh gas volume, and the bosh gas composition of the tuyere raceway zone can be determined according to the raw fuel parameters, the blast parameters, and the gas injection parameters, thereby determining the theoretical combustion temperature of gas.

In one embodiment of the present application, Step S120 includes: determining, based on the raw fuel parameters, the blast parameters, and the gas injection parameters, a volume of gas participating in chemical reactions, a volume of gas not participating in chemical reactions, and a volume of gas generated by chemical reactions in the tuyere raceway zone; calculating heat released by chemical reactions in the tuyere raceway zone based on the volume of gas participating in chemical reactions, and calculating the total heat input according to sensible heat of coke, sensible heat of blast, sensible heat of injected gas, and the heat released by chemical reactions, wherein the sensible heat of coke is determined based on the raw fuel parameters, the sensible heat of blast is determined based on the blast parameters, and the sensible heat of injected gas is determined based on the gas injection parameters; calculating the bosh gas volume and the bosh gas composition according to the volume of gas generated by chemical reactions and the volume of gas not participating in chemical reactions; and performing an iterative solution for the theoretical combustion temperature of gas based on the bosh gas volume, the bosh gas composition, and the total heat input until a second convergence condition is satisfied, thereby obtaining the theoretical combustion temperature of gas.

In this embodiment, reference is made to Table 1, which is a table of chemical reactions and corresponding thermal effects in the tuyere raceway zone according to a specific embodiment of the present application. As shown in Table 1, in the tuyere raceway zone, injected gas, injected pulverized coal, and coke, including elemental carbon, or CO₂, or H₂O (water), or CH₄, or chemical species CₓH_{γ}OₘNₙ, undergo combustion and other chemical reactions upon contact with oxygen or other substances, and are ultimately converted into CO, H₂, and N₂. Different chemical reactions correspond to different thermal effects (reaction enthalpies).

**Table 1**

| serial number | reaction equation | thermal effects(kJ/mol) |
|---|---|---|
| 1 | C+CO₂=2CO | ΔH_{C1} |
| 2 | C+H₂O=H₂+CO | ΔH_{C2} |
| 3 | CH₄+0.5O₂=CO+H₂ | ΔH_{CH4} |
| 4 | C+0.5O₂=CO | ΔH_{C3} |
| 5 | CₓH_{y}OₘNₙ+O₂=CO+H₂+N₂ | ΔH_{C4} |

Therefore, the calculation method for the heat released by chemical reactions in the tuyere raceway is as follows:
$Q 2 = a Δ HC 1 + b Δ HC 2 + c Δ HCH 4 + d Δ HC 3 + e Δ HC 4$

Wherein Q₂ represents the heat released by chemical reactions in the tuyere raceway; a, b, c, d, and e respectively represent reaction participation amounts corresponding to reactions 1-5 in Table 1; and Δ*H*_{*C*1}*,* Δ*H*_{*C*2}*,* Δ*H*_{CH4}*,* Δ*H*_{*C*3} and Δ*H*_{*C*4} respectively represent heat effects corresponding to reactions 1-5 in Table 1.The values of a, b, and c depend on volume amounts of CO₂, H₂O, and CH₄ in the injected gas, and the values of d and e depend on a total amount of oxygen entering the blast furnace. It should be noted that, when calculating the heat released by chemical reactions in the tuyere raceway, O₂ preferentially reacts with CH₄ and gases such as CₓH_{y}OₘNₙ, and finally reacts with solid carbon C. The combustion priority among gas, pulverized coal, coke, and oxygen is: gas > pulverized coal > coke, and a burnout rate of pulverized coal is 70-84%.

Therefore, when calculating the heat released by chemical reactions in the tuyere raceway, a volume of gas participating in chemical reactions in the tuyere raceway is first determined, including a CO₂ volume, an H₂O volume, a CH₄ volume, a CₓH_{y}OₘNₙ volume, and an O₂ volume. Specifically, a CO₂ volume, an H₂O volume, a CH₄ volume, and a CₓH_{y}OₘNₙ volume in the injected gas are determined according to a CO₂ proportion, an H₂O proportion, a CH₄ proportion, a CₓH_{y}OₘNₙ proportion, and a gas injection amount in the gas injection composition, wherein the gas injection parameter comprises the gas injection composition and the gas injection amount. An O₂ volume in the injected gas is determined according to an O₂ proportion in the gas injection composition and the gas injection amount. An O₂ volume in blast is determined according to an O₂ proportion in the blast composition and a blast amount, wherein the blast parameter comprises the blast composition and the blast amount. An O₂ volume in coke is determined according to an O₂ proportion in the coke composition and a coke amount, and an O₂ volume in pulverized coal is determined according to an O₂ proportion in the pulverized coal composition and a pulverized coal amount, wherein the raw fuel parameter comprises the coke composition, the coke amount, the pulverized coal composition, and the pulverized coal amount. A total amount of oxygen entering the blast furnace is calculated according to the O₂ volume in the injected gas, the O₂ volume in blast, the O₂ volume in coke, and the O₂ volume in pulverized coal, and a calculation method thereof is as follows:
${V}_{t} = {V}_{Blast}^{0} + {V}_{Gas}^{0} + {v}_{Coke}^{0} + {V}_{Coal}^{0}$
wherein V₁ represents the total amount of oxygen entering the blast furnace, ${V}_{blast}^{0}$ represents the volume of O₂ in the blast, ${V}_{Gas}^{0}$ represents the volume of O₂ in the injected gas, ${V}_{coke}^{0}$ represents the volume of O₂ in the coke, and ${V}_{coal}^{0}$ represents the volume of O₂ in the pulverized coal.

After obtaining the heat released by the chemical reactions in the tuyere raceway zone, the total heat input of the tuyere raceway zone is calculated based on the sensible heat of the coke, the sensible heat of the blast, the sensible heat of the injected gas, and the heat released by the chemical reactions, and the calculation is expressed as follows:
$Q = {Q}_{1} + {Q}_{2} + {Q}_{3} + {Q}_{4}$

Wherein Q represents the total heat input of the tuyere raceway,*Q*₁ represents blast sensible heat, *Q*₂ represents heat released by chemical reactions in the tuyere raceway, *Q*₃ represents gas sensible heat, and *Q*₄ represents coke sensible heat.The blast sensible heat is determined based on the blast parameter, the gas sensible heat is determined based on the gas injection parameter, and the coke sensible heat is determined based on the raw fuel parameter.

Bosh gas at the tuyere raceway comprises gas generated by chemical reactions and gas not participating in chemical reactions. A volume of gas generated by chemical reactions in the tuyere raceway, including a CO volume and an H₂ volume, can be determined according to a volume of gas participating in chemical reactions in the tuyere raceway and reaction equations listed in Table 1.Since N₂ is contained in pulverized coal, injected gas, and blast, and it can be seen from Table 1 that gas participating in chemical reactions does not include N₂, an N₂ volume in pulverized coal, an N₂ volume in injected gas, and an N₂ volume in blast can be determined as a volume of gas not participating in chemical reactions.Specifically, an N₂ volume in pulverized coal is determined according to an N₂ proportion in a pulverized coal composition and a pulverized coal amount; an N₂ volume in injected gas is determined according to an N₂ proportion in a gas injection composition and a gas injection amount; and an N₂ volume in blast is determined according to an N₂ proportion in a blast composition and a blast amount.

Then, a bosh gas volume is calculated according to the volume of gas generated by chemical reactions and the volume of gas not participating in chemical reactions, and a calculation method thereof is as follows: ${V}_{G} = {V}_{Coke}^{CO} + {V}_{Coal}^{CO} + {V}_{Gas}^{CO} + {V}_{Blast}^{CO} + {V}_{Coal}^{{H}_{2}} + {V}_{Gas}^{{H}_{2}} + {V}_{Blast}^{{H}_{2}} + {V}_{Coal}^{{N}_{2}} + {V}_{Gas}^{{N}_{2}} + {V}_{Blast}^{{N}_{2}}$

Wherein V_{G} represents the bosh gas volume; ${V}_{Coke}^{CO}$ represents a CO volume generated by reaction of coke in the tuyere raceway; ${V}_{Coal}^{CO}$ represents a CO volume generated by reaction of pulverized coal in the tuyere raceway; ${V}_{Gas}^{CO}$ represents a CO volume generated by reaction of injected gas in the tuyere raceway; ${V}_{Blast}^{CO}$ represents a CO volume generated by reaction of water in blast in the tuyere raceway; ${V}_{Coal}^{{H}_{2}}$ represents an H₂ volume generated by reaction of hydrogen in moisture and volatile matter of pulverized coal in the tuyere raceway; ${V}_{Gas}^{{H}_{2}}$ represents an H₂ volume generated by reaction of water in blast in the tuyere raceway; ${V}_{Blast}^{{H}_{2}}$ represents an H₂ volume generated by reaction of water and hydrogen-containing gases (such as methane, ethane, ethylene, and the like) in injected gas in the tuyere raceway; ${V}_{Coal}^{{N}_{2}}$ represents an N₂ volume in pulverized coal; ${V}_{Gas}^{{N}_{2}}$ represents an N₂ volume in injected gas; and ${V}_{Blast}^{{N}_{2}}$ represents an N₂ volume in blast.Illustratively, ${V}_{Coal}^{CO}$ includes a CO volume generated by reaction of fixed carbon, volatile carbon, and moisture in pulverized coal, and ${V}_{Coal}^{{N}_{2}}$ includes an N₂ volume in volatile matter of pulverized coal and a carrier gas of pulverized coal.

As can be seen from Equation (4), bosh gas comprises CO, H₂, and N₂. A CO volume, an H₂ volume, and an N₂ volume in bosh gas are determined according to the volume of gas generated by chemical reactions and the volume of gas not participating in chemical reactions. A CO proportion in bosh gas is calculated according to the CO volume in bosh gas and the bosh gas volume, an H₂ proportion in bosh gas is calculated according to the H₂ volume in bosh gas and the bosh gas volume, and an N₂ proportion in bosh gas is calculated according to the N₂ volume in bosh gas and the bosh gas volume, so as to use the CO proportion, the H₂ proportion, and the N₂ proportion in bosh gas as the bosh gas composition.

After the total heat input, the bosh gas volume, and the bosh gas composition are obtained, an iterative solution of the theoretical combustion temperature of gas is performed based on the bosh gas volume, the bosh gas composition, and the total heat input until the second convergence condition is satisfied, so as to obtain the theoretical combustion temperature of gas.Illustratively, the second convergence condition may be that a number of iterations of the iterative solution of the theoretical combustion temperature of gas reaches a second preset iteration number, or that a theoretical combustion temperature iteration difference is less than or equal to a second preset temperature difference threshold, or other convergence conditions, which are not limited herein.By using a sum of the coke sensible heat, the blast sensible heat, the gas sensible heat, and the heat released by chemical reactions as the total heat input, released heat is comprehensively considered, thereby improving accuracy of calculation of the theoretical combustion temperature of gas.

In one embodiment of the present application, before calculating the total heat input according to the coke sensible heat, the blast sensible heat, the gas sensible heat, and the heat released by chemical reactions in the tuyere raceway, the method comprises: determining a coke specific heat capacity according to a preset coke temperature and a coke composition, so as to calculate the coke sensible heat according to the preset coke temperature, the coke specific heat capacity, and a coke amount, wherein the raw fuel parameter comprises the coke composition and the coke amount; determining a blast specific heat capacity according to a blast temperature and a blast composition, so as to calculate the blast sensible heat according to the blast specific heat capacity, a blast amount, and the blast temperature, wherein the blast parameter comprises the blast composition, the blast temperature, and the blast amount; and determining a gas injection specific heat capacity according to a preset gas injection temperature and a gas injection composition, so as to calculate the gas sensible heat according to the preset gas injection temperature, the gas injection specific heat capacity, and a gas injection amount, wherein the gas injection parameter comprises the gas injection composition and the gas injection amount.

In this embodiment, blast, coke, and injected gas are each composed of different substances. Specific heat capacities of different substances are different, and a specific heat capacity of the same substance varies at different temperatures. Therefore, a relationship between specific heat capacity and temperature of different substances may be established in advance.

When calculating the blast sensible heat, first, specific heat capacities of respective substances in blast at the blast temperature are determined according to the preset relationships between specific heat capacity and temperature of different substances and the blast temperature in the blast parameter. Then, a blast specific heat capacity is calculated according to the specific heat capacities of the respective substances in blast at the blast temperature and proportions of the respective substances in the blast composition of the blast parameter. Finally, the blast sensible heat is calculated according to the blast specific heat capacity, the blast temperature, and the blast amount in the blast parameter, and a calculation method thereof is as follows:
${Q}_{1} = {C}_{b} {V}_{b} {T}_{b}$

Wherein *Q*₁ represents the blast sensible heat, *C_{b}* represents the blast specific heat capacity, *V_{b}* represents the blast amount, and *T_{b}* represents the blast temperature.

When calculating the gas sensible heat, first, specific heat capacities of respective substances in injected gas at the preset gas injection temperature are determined according to preset relationships between specific heat capacity and temperature of different substances and the preset gas injection temperature. Then, a gas injection specific heat capacity is calculated according to the specific heat capacities of the respective substances in injected gas at the preset gas injection temperature and proportions of the respective substances in the gas injection composition of the gas injection parameter. Finally, the gas sensible heat is calculated according to the gas injection specific heat capacity, the preset gas injection temperature, and the gas injection amount in the gas injection parameter, and a calculation method thereof is as follows:
${Q}_{3} = {C}_{gi} {V}_{gi} {T}_{gi}$

Wherein *Q*₃ represents the gas sensible heat, *C_{gi}* represents the gas injection specific heat capacity, *V_{gi}* represents the gas injection amount, and *T_{gi}* represents the preset gas injection temperature.

When calculating the coke sensible heat, first, specific heat capacities of respective substances in coke at the preset coke temperature are determined according to preset relationships between specific heat capacity and temperature of different substances and the preset coke temperature. Then, a coke specific heat capacity is calculated according to the specific heat capacities of the respective substances in coke at the preset coke temperature and proportions of the respective substances in the coke composition of the raw fuel parameter. Finally, the coke sensible heat is calculated according to the coke specific heat capacity, the preset coke temperature, and the coke amount in the raw fuel parameter, and a calculation method thereof is as follows:
${Q}_{4} = {C}_{coke} {m}_{coke} {T}_{coke}$

Wherein *Q*₄ represents the coke sensible heat, *C_{coke}* represents the coke specific heat capacity, *m_{coke}* represents the coke amount, and *T_{coke}* represents the preset coke temperature. Illustratively, the preset coke temperature may be any temperature value in a range of 1400-1500°C, or other temperature values, which are not limited herein.

In this embodiment, influences of temperature and proportions of respective substances on specific heat capacities of blast, gas, and coke are taken into consideration. By determining specific heat capacities of blast, gas, and coke based on temperature and proportions of respective substances, accuracy of the specific heat capacities of blast, gas, and coke is improved, thereby improving accuracy of calculation of the blast sensible heat, the gas sensible heat, and the coke sensible heat.

In one embodiment of the present application, an iterative solution of the theoretical combustion temperature of gas is performed based on the bosh gas volume, the bosh gas composition, and the total heat input until the second convergence condition is satisfied, so as to obtain the theoretical combustion temperature of gas, comprising: determining an initial bosh gas specific heat capacity of the tuyere raceway according to the bosh gas composition and a preset theoretical combustion temperature of gas, and calculating an initial theoretical combustion temperature of gas according to the total heat input, the bosh gas volume, and the initial bosh gas specific heat capacity; using the initial theoretical combustion temperature of gas as a pre-iteration theoretical combustion temperature of gas, determining an iterative bosh gas specific heat capacity of the tuyere raceway according to the bosh gas composition and the pre-iteration theoretical combustion temperature of gas, and calculating a post-iteration theoretical combustion temperature of gas according to the total heat input, the bosh gas volume, and the iterative bosh gas specific heat capacity; calculating a difference between the pre-iteration theoretical combustion temperature of gas and the post-iteration theoretical combustion temperature of gas to obtain a theoretical combustion temperature iteration difference; if the theoretical combustion temperature iteration difference is less than or equal to a second preset temperature difference threshold, determining that the second convergence condition is satisfied, and using the post-iteration theoretical combustion temperature of gas as the theoretical combustion temperature of gas; and if the theoretical combustion temperature iteration difference is greater than the second preset temperature difference threshold, performing the iterative solution of the theoretical combustion temperature of gas based on the bosh gas volume, the bosh gas composition, the total heat input, and the post-iteration theoretical combustion temperature of gas until the second convergence condition is satisfied, so as to obtain the theoretical combustion temperature of gas.

In this embodiment, a calculation formula of the theoretical combustion temperature of gas is as follows:
${T}_{f} = \frac{Q}{{V}_{G} ⋅ {C}_{G}}$

Wherein *T_{f}* represents the theoretical combustion temperature of gas, Q represents the total heat input, *V_{G}* represents the bosh gas volume, and *C_{G}* represents the bosh gas specific heat capacity.

Based on this, an iterative solution process of the theoretical combustion temperature of gas may be as follows:
1.A temperature value is preset as a preset theoretical combustion temperature of gas. According to preset relationships between specific heat capacity and temperature of different substances and the preset theoretical combustion temperature of gas, specific heat capacities of respective substances in bosh gas at the preset theoretical combustion temperature of gas are determined. According to the specific heat capacities of the respective substances in bosh gas at the preset theoretical combustion temperature of gas and proportions of the respective substances in the bosh gas composition, an initial bosh gas specific heat capacity is calculated. The total heat input, the bosh gas volume, and the initial bosh gas specific heat capacity are substituted into Equation (8) to calculate an initial theoretical combustion temperature of gas, and the initial theoretical combustion temperature of gas is used as a pre-iteration theoretical combustion temperature of gas.
2.According to the preset relationships between specific heat capacity and temperature of different substances and the pre-iteration theoretical combustion temperature of gas, specific heat capacities of respective substances in bosh gas at the pre-iteration theoretical combustion temperature of gas are determined. According to the specific heat capacities of the respective substances in bosh gas at the pre-iteration theoretical combustion temperature of gas and proportions of the respective substances in the bosh gas composition, an iterative bosh gas specific heat capacity is calculated. The total heat input, the bosh gas volume, and the iterative bosh gas specific heat capacity are substituted into Equation (8) to calculate a post-iteration theoretical combustion temperature of gas.
3.A difference between the pre-iteration theoretical combustion temperature of gas and the post-iteration theoretical combustion temperature of gas is calculated as a theoretical combustion temperature iteration difference, and the theoretical combustion temperature iteration difference is compared with a second preset temperature difference threshold.
4.If the theoretical combustion temperature iteration difference is less than or equal to the second preset temperature difference threshold, it is determined that the second convergence condition is satisfied, and the post-iteration theoretical combustion temperature of gas is used as the theoretical combustion temperature of gas. Otherwise, the post-iteration theoretical combustion temperature of gas is used as a new pre-iteration theoretical combustion temperature of gas, and steps 2-3 are repeated until the second convergence condition is satisfied, and a post-iteration theoretical combustion temperature of gas obtained in a last iterative solution is used as the theoretical combustion temperature of gas.

By performing the iterative solution of the theoretical combustion temperature of gas, accuracy of the theoretical combustion temperature of gas is improved, thereby further improving accuracy of molten iron temperature prediction.

Step S130: determining a direct reduction degree of iron ore according to the top gas parameter, the slag-iron parameter, the bosh gas volume, and the bosh gas composition.

In one embodiment of the present application, since direct reduction of elements such as Fe, Si, Mn, S, and P in molten iron consumes heat, reduction of these elements affects the molten iron temperature. Based on this, the direct reduction degree of iron ore is determined, and may be calculated according to the top gas parameter, the slag-iron parameter, the bosh gas volume, and the bosh gas composition.

In one embodiment of the present application, Step S130 comprises: determining a CO amount in bosh gas based on the bosh gas volume and the bosh gas composition; determining a total amount of CO and CO₂ in top gas based on the top gas volume and the top gas composition; determining a total amount of CO generated by direct reduction according to the CO amount in bosh gas and the total amount of CO and CO₂ in top gas, wherein the top gas parameter comprises the top gas volume and the top gas composition; determining a mass of trace elements in molten iron and a mass of iron in molten iron based on a molten iron composition and a molten iron yield; determining a CO amount generated by direct reduction of trace element oxides based on the mass of trace elements in molten iron; determining a CO amount generated by direct reduction of iron ore according to the total amount of CO generated by direct reduction and the CO amount generated by direct reduction of trace element oxides, wherein the slag-iron parameter comprises the molten iron composition and the molten iron yield; and calculating the direct reduction degree of iron ore based on the CO amount generated by direct reduction of iron ore, the mass of iron in molten iron, and a preset mass of iron in a hot-pressed iron block.

In this embodiment, a CO volume (i.e., a CO amount) in bosh gas is calculated according to a CO proportion in the bosh gas composition and the bosh gas volume; a CO volume in top gas is calculated according to a CO proportion in the top gas composition and the top gas volume; and a CO₂ volume in top gas is calculated according to a CO₂ proportion in the top gas composition and the top gas volume. A sum of the CO volume and the CO₂ volume in top gas is calculated as the total amount of CO and CO₂ in top gas. A difference between the total amount of CO and CO₂ in top gas and the CO amount in bosh gas is calculated as the total amount of CO generated by direct reduction, and a calculation method thereof is as follows:
${V}_{1} = {V}_{\left.CO\right| CO 2} - {V}_{COb}$

Wherein *V*₁ represents a total amount of CO generated by direct reduction, *V*_{*CO* | *CO*2} represents a total amount of CO and CO₂ in top gas, and *V_{COb}* represents a CO amount in bosh gas.

It should be understood that, during a direct reduction reaction, iron ore undergoes direct reduction to generate reduced products of Fe, and trace element oxides undergo direct reduction to generate reduced products of trace elements. Trace elements in molten iron include elements such as Si, Mn, P, Ti, and S, and correspondingly, trace element oxides include oxides of Si, Mn, P, Ti, and S, and reduced products of trace elements include reduced products of Si, Mn, P, Ti, and S. Therefore, masses of trace elements in molten iron, such as a mass of Si, a mass of Mn, a mass of P, a mass of Ti, and a mass of S, may be determined based on proportions of trace elements in a molten iron composition, such as a Si proportion, an Mn proportion, a P proportion, a Ti proportion, and a S proportion, and a molten iron yield. Accordingly, a CO amount generated by direct reduction of trace element oxides such as oxides of Si, Mn, P, Ti, and S may be calculated based on the masses of trace elements Si, Mn, P, Ti, and S in molten iron, and a calculation method thereof is as follows:
${V}_{2} = 22.4 \times \left(2\times \frac{{m}_{\left[Si\right]}}{28}+\frac{{m}_{\left[Mn\right]}}{55}+5\times \frac{{m}_{\left[p\right]}}{2 \times 31}+2\times \frac{{m}_{\left[Ti\right]}}{48}+\frac{{m}_{\left[s\right]}}{32}\right)$

Wherein *V*₂ represents a CO amount generated by direct reduction of trace element oxides such as Si, Mn, P, Ti, and S; *m*_{[*Si*]} represents a mass of Si in molten iron; *m*_{[*Mn*]} represents a mass of Mn in molten iron; *m*_{[*p*]} represents a mass of P in molten iron; *m*_{[*Ti*]} represents a mass of Ti in molten iron; and *m*_{[*s*]} represents a mass of S in molten iron.

A difference between the total amount of CO generated by direct reduction and the CO amount generated by direct reduction of trace element oxides is calculated as a CO amount generated by direct reduction of iron ore, and a calculation method thereof is as follows:${V}_{3} = {V}_{1} - {V}_{2}$

Wherein *V*₃ represents a CO amount generated by direct reduction of iron ore, *V₁* represents the total amount of CO generated by direct reduction, and *V*₂ represents the CO amount generated by direct reduction of trace element oxides.

A mass of iron in molten iron is determined based on an iron proportion in a molten iron composition and a molten iron yield. Then, the direct reduction degree of iron ore is calculated based on the CO amount generated by direct reduction of iron ore, the mass of iron in molten iron, and a mass of iron in a preset hot briquetted iron block, and a calculation method thereof is as follows:$Rd = \left(\frac{{V}_{3}}{12}\times 56\right) / \left({m}_{\left[Fe\right]}-{m}_{l}\right)$

Wherein Rd represents a direct reduction degree of iron ore, *V*₃ represents a CO amount generated by direct reduction of iron ore, *m*_{[*Fe*]} represents a mass of iron in molten iron, and *mₗ* represents an iron content of a hot briquetted iron used in a blast furnace, that is, a mass of iron in a preset hot briquetted iron block.

In this embodiment, reducibility of trace element oxides is taken into account. By determining a total CO amount generated by direct reduction and a CO amount generated by direct reduction of trace element oxides, the CO amount generated by direct reduction of iron ore is further determined, and the direct reduction degree of iron ore is calculated based on the CO amount generated by direct reduction of iron ore, thereby improving accuracy of the direct reduction degree of iron ore.

Step S140: determination of gas-liquid heat exchange coefficient.Step S140 includes determining a gas-liquid heat exchange coefficient based on a theoretical combustion temperature of gas, a belly gas volume, a belly gas composition, slag-iron parameters, and a post-direct-reduction gas composition at a direct reduction position.

### Embodiment 1: determining post-direct-reduction gas composition based on top gas

In one embodiment of the present application, since heat exchange exists between molten iron and gas, accuracy of molten iron temperature prediction is also related to the gas-liquid heat exchange coefficient. Accordingly, the gas-liquid heat exchange coefficient may be calculated based on the theoretical combustion temperature of gas, the belly gas volume, the belly gas composition, the slag-iron parameters, and the post-direct-reduction gas composition at the direct reduction position. Since a post-direct-reduction gas volume is equal to a top gas volume, a CO volume in post-direct-reduction gas is equal to a sum of CO and CO₂ volumes in the top gas, an H₂ volume in post-direct-reduction gas is equal to a sum of H₂ and H₂O volumes in the top gas or an H₂ volume in the belly gas, and an N₂ volume in post-direct-reduction gas is equal to an N₂ volume in the top gas or an N₂ volume in the belly gas, the post-direct-reduction gas composition may be determined based on the top gas parameters, or based on the top gas parameters together with the belly gas volume and the belly gas composition.

In one embodiment, before determining the gas-liquid heat exchange coefficient, the method includes: determining a total amount of CO and CO₂, a total amount of H₂O and H₂, and an amount of N₂ in top gas based on a top gas volume and a top gas composition, wherein the top gas parameters include the top gas volume and the top gas composition; taking the top gas volume as a post-direct-reduction gas volume, taking the total amount of CO and CO₂ in the top gas as a CO amount in post-direct-reduction gas, taking the total amount of H₂O and H₂ in the top gas as an H₂ amount in post-direct-reduction gas, and taking the amount of N₂ in the top gas as an N₂ amount in post-direct-reduction gas; and determining the post-direct-reduction gas composition based on the post-direct-reduction gas volume, the CO amount, the H₂ amount, and the N₂ amount in the post-direct-reduction gas.

In this embodiment, the post-direct-reduction gas composition can be accurately determined based on a relationship between the top gas and the post-direct-reduction gas.

Specifically, an H₂ volume in the top gas is calculated based on an H₂ proportion in the top gas composition and the top gas volume, and an H₂O volume in the top gas is calculated based on an H₂O proportion in the top gas composition and the top gas volume, and a sum thereof is taken as a total amount of H₂O and H₂ in the top gas. A CO volume and a CO₂ volume in the top gas are calculated respectively based on CO and CO₂ proportions in the top gas composition and the top gas volume, and a sum thereof is taken as a total amount of CO and CO₂ in the top gas. An N₂ volume in the top gas is calculated based on an N₂ proportion in the top gas composition and the top gas volume.

Then, the top gas volume is taken as the post-direct-reduction gas volume, the total amount of CO and CO₂ in the top gas is taken as the CO amount in the post-direct-reduction gas, the total amount of H₂O and H₂ in the top gas is taken as the H₂ amount in the post-direct-reduction gas, and the amount of N₂ in the top gas is taken as the N₂ amount in the post-direct-reduction gas.

Finally, an H₂ proportion in the gas after direct reduction is determined based on an amount of H₂ in the gas after direct reduction and a total gas volume after direct reduction; a CO proportion in the gas after direct reduction is determined based on an amount of CO in the gas after direct reduction and the total gas volume after direct reduction; and an N₂ proportion in the gas after direct reduction is determined based on an amount of N₂ in the gas after direct reduction and the total gas volume after direct reduction, such that the H₂ proportion, the CO proportion, and the N₂ proportion in the gas after direct reduction are taken as the gas composition after direct reduction.

### Embodiment 2: determining post-direct-reduction gas composition based on top gas and belly gas

In another embodiment, before determining the gas-liquid heat exchange coefficient, the method includes: determining a total amount of CO and CO₂ in the top gas based on the top gas volume and the top gas composition; determining an H₂ amount and an N₂ amount in the belly gas based on the belly gas volume and the belly gas composition; taking the top gas volume as the post-direct-reduction gas volume, taking the total amount of CO and CO₂ in the top gas as the CO amount in the post-direct-reduction gas, taking the H₂ amount in the belly gas as the H₂ amount in the post-direct-reduction gas, and taking the N₂ amount in the belly gas as the N₂ amount in the post-direct-reduction gas; and determining the post-direct-reduction gas composition based on the post-direct-reduction gas volume, the CO amount, the H₂ amount, and the N₂ amount.

In this embodiment, the post-direct-reduction gas composition can be accurately determined based on relationships among the top gas, the belly gas, and the post-direct-reduction gas.

First, a CO volume in the top gas is calculated based on a CO proportion in the top gas composition and a top-gas flow rate, and a CO₂ volume in the top gas is calculated based on a CO₂ proportion in the top gas composition and the top-gas flow rate, wherein a sum of the CO volume and the CO₂ volume is taken as a total amount of CO and CO₂ in the top gas; an H₂ volume in the bosh gas is determined based on an H₂ proportion in the bosh-gas composition and a bosh-gas flow rate, which is taken as an amount of H₂ in the bosh gas; and an N₂ volume in the bosh gas is determined based on an N₂ proportion in the bosh-gas composition and the bosh-gas flow rate, which is taken as an amount of N₂ in the bosh gas.

Then, the top-gas flow rate is taken as a gas flow rate after direct reduction, the total amount of CO and CO₂ in the top gas is taken as an amount of CO in the gas after direct reduction, the amount of H₂ in the bosh gas is taken as an amount of H₂ in the gas after direct reduction, and the amount of N₂ in the bosh gas is taken as an amount of N₂ in the gas after direct reduction.

Finally, an H₂ proportion in the gas after direct reduction is determined based on the amount of H₂ in the gas after direct reduction and the gas flow rate after direct reduction; a CO proportion in the gas after direct reduction is determined based on the amount of CO in the gas after direct reduction and the gas flow rate after direct reduction; and an N₂ proportion in the gas after direct reduction is determined based on the amount of N₂ in the gas after direct reduction and the gas flow rate after direct reduction, such that the H₂ proportion, the CO proportion, and the N₂ proportion in the gas after direct reduction are taken as a gas composition after direct reduction.

In one embodiment, Step S140 further includes: performing interpolation calculation on the belly gas composition and the post-direct-reduction gas composition to obtain a dripping zone gas composition; determining a gas-liquid heat exchange coefficient of a tuyere raceway based on a slag-iron equivalent particle diameter, a gas thermal conductivity of the tuyere raceway, and a Nusselt number of tuyere raceway, wherein the gas thermal conductivity and the Nusselt number of the tuyere raceway are both determined based on the belly gas composition and the theoretical combustion temperature of gas, and the slag-iron equivalent particle diameter is determined based on the slag-iron parameters; determining a gas-liquid heat exchange coefficient at the direct reduction position based on the slag-iron equivalent particle diameter, a gas thermal conductivity at the direct reduction position, and a Nusselt number at the direct reduction position, wherein the gas thermal conductivity and the Nusselt number at the direct reduction position are both determined based on the post-direct-reduction gas composition and the theoretical combustion temperature of gas; and determining a gas-liquid heat exchange coefficient of the dripping zone based on the slag-iron equivalent particle diameter, a gas thermal conductivity of the dripping zone, and a Nusselt number of the dripping zone, wherein the gas thermal conductivity and the Nusselt number of the dripping zone are both determined based on the dripping zone gas composition and the theoretical combustion temperature of gas.

In this embodiment, the dripping zone is located between the tuyere raceway and the direct reduction position. Since gas compositions vary at different heights within the dripping zone, and gas-liquid heat exchange coefficients corresponding to different gas compositions are also different, gas-liquid heat exchange coefficients at different heights in the dripping zone are determined to further improve accuracy of molten iron temperature prediction.

Illustratively, the gas-liquid heat exchange coefficient may be determined based on a correction factor, the gas thermal conductivity, the slag-iron equivalent particle diameter, and the Nusselt number. A calculation method thereof is as follows:
${h}_{g - l} = γ ⋅ {k}_{g} ⋅ Nu / {d}_{l}$

Wherein *h_{g-l}* represents a gas-liquid heat exchange coefficient, *γ* represents a correction factor, *k_{g}* represents a gas thermal conductivity, *Nu* represents a Nusselt number, and *dₗ* represents a slag-iron equivalent particle diameter.

The Nusselt number is calculated as follows:
$Nu = 2.0 + 0.6 {\left(9{R}_{{e}_{p}}\right)}^{1 / 2} {Pr}^{1 / 3}$

Wherein *Nu* represents a Nusselt number, *Reₚ* represents a Reynolds number, and *Pr* represents a Prandtl number.

The Reynolds number is calculated as follows:
${Re}_{p} = {ρ}_{g} {d}_{s} {u}_{g} / {μ}_{g}$

Wherein *Reₚ* represents a Reynolds number, *ρ_{g}* represents gas density, *dₛ* represents a characteristic length, *u_{g}* represents gas flow velocity, and *µ_{g}* represents gas viscosity.

The Prandtl number is calculated as follows:
$Pr = {u}_{g} {C}_{G} / {k}_{g}$

Wherein *Pr* represents the Prandtl number, *u_{g}* represents gas flow velocity, *C_{G}* represents gas specific heat capacity, and *k_{g}* represents gas thermal conductivity coefficient.

It should be noted that the gas thermal conductivity coefficient, gas density, gas viscosity, and gas specific heat capacity are all related to the gas composition and gas temperature. In particular, the relationship between the gas thermal conductivity coefficient and the gas composition is as follows:
${k}_{g} = {m}_{CO 1} {k}_{CO} + {m}_{H 21} {k}_{H 2} + {m}_{N 21} {k}_{N 2}$

Wherein *k_{g}* represents the gas thermal conductivity coefficient, *m*_{*CO*1} represents the CO proportion in the gas, *m*_{*H*21} represents the H₂ proportion in the gas, *m*_{*N*21} represents the N₂ proportion in the gas, *k_{CO}* represents the thermal conductivity coefficient of CO, *k*_{*H*2} represents the thermal conductivity coefficient of H₂, and *k*_{*N*2} represents the thermal conductivity coefficient of N₂.

The relationship between the gas density and the gas composition is as follows:
${ρ}_{g} = {m}_{CO 1} {ρ}_{CO} + {m}_{H 21} {ρ}_{H 2} + {m}_{N 21} {ρ}_{N 2}$

Wherein *ρ_{g}* represents the gas density, *m*_{*CO*1} represents the CO proportion in the gas, *m*_{*H*21} represents the H₂ proportion in the gas, *m*_{*N*21} represents the N₂ proportion in the gas, *ρ_{CO}* represents the density of CO, *ρ*_{*H*2} represents the density of H₂, and *ρ*_{*N*2} represents the density of N₂.

The relationship between the gas viscosity and the gas composition is as follows:
${μ}_{g} = {m}_{CO 1} {μ}_{CO} + {m}_{H 21} {μ}_{H 2} + {m}_{N 21} {μ}_{N 2}$
wherein *µ_{g}* is gas viscosity, *m*_{*CO*1} is a proportion of CO in the gas, *m*_{*H*21} is a proportion of H₂ in the gas, *m*_{*N*21} is a proportion of N₂ in the gas, *µ_{CO}* is viscosity of CO, *µ*_{*H*2} is viscosity of H₂, and *µ*_{*N*2} is viscosity of N₂.

The relationship between gas specific heat capacity and gas composition is as follows:
${C}_{G} = {m}_{CO 1} {C}_{CO} + {m}_{H 21} {C}_{H 2} + {m}_{N 21} {C}_{N 2}$
wherein *C_{G}* is gas specific heat capacity, *m*_{*CO*1} is a proportion of CO in the gas, *m*_{*H*21} is a proportion of H₂ in the gas, *m*_{*N*21} is a proportion of N₂ in the gas, *C_{CO}* is specific heat capacity of CO, *C*_{*H*2} is specific heat capacity of H₂, and *C*_{*N*2} is specific heat capacity of N₂.

Illustratively, a determination procedure of the gas-liquid heat exchange coefficient in the tuyere raceway may include:
determining the specific heat capacity of CO, the specific heat capacity of H₂, and the specific heat capacity of N₂ according to the theoretical combustion temperature of gas and a preset correspondence relationship between specific heat capacity and temperature of each substance; substituting the specific heat capacity of CO, the specific heat capacity of H₂, and the specific heat capacity of N₂, together with proportions of CO, H₂, and N₂ in the bosh gas composition, into Equation (20) to obtain gas specific heat capacity of the tuyere raceway; determining viscosity of CO, viscosity of H₂, and viscosity of N₂ according to the theoretical combustion temperature of gas and a preset correspondence relationship between viscosity and temperature of each substance; substituting the viscosity of CO, the viscosity of H₂, and the viscosity of N₂, together with proportions of CO, H₂, and N₂ in the bosh gas composition, into Equation (19) to obtain gas viscosity of the tuyere raceway; determining density of CO, density of H₂, and density of N₂ according to the theoretical combustion temperature of gas and a preset correspondence relationship between density and temperature of each substance; substituting the density of CO, the density of H₂, and the density of N₂, together with proportions of CO, H₂, and N₂ in the bosh gas composition, into Equation (18) to obtain gas density of the tuyere raceway; determining thermal conductivity of CO, thermal conductivity of H₂, and thermal conductivity of N2 according to the theoretical combustion temperature of gas and a preset correspondence relationship between thermal conductivity and temperature of each substance; substituting the thermal conductivity of CO, the thermal conductivity of H2, and the thermal conductivity of N₂, together with proportions of CO, H₂, and N₂ in the bosh gas composition, into Equation (17) to obtain gas thermal conductivity of the tuyere raceway. The gas thermal conductivity and the gas specific heat capacity of the tuyere raceway are substituted into Equation (16) to obtain the Prandtl number of the tuyere raceway, and the gas density and the gas viscosity of the tuyere raceway are substituted into Equation (15) to obtain the Reynolds number of the tuyere raceway, wherein gas flow velocity is obtained by dividing gas volume by a blast furnace cross-sectional area at a calculated position, the cross-sectional area being determined by blast furnace profile structure, a characteristic length is slag-iron equivalent particle size, and the slag-iron equivalent particle size is determined based on molten iron density, liquid slag density, blast furnace profile structure, and slag amount in the slag-iron parameter; the Prandtl number and the Reynolds number of the tuyere raceway are substituted into Equation (14) to obtain the Nusselt number of the tuyere raceway; and the gas thermal conductivity and the Nusselt number of the tuyere raceway are substituted into Equation (13) to obtain the gas-liquid heat exchange coefficient of the tuyere raceway, wherein a correction coefficient may be preset and may be any value in a range of 0.2-0.5, or other values, without limitation herein.

Correspondingly, according to the above method, a gas-liquid heat exchange coefficient at a direct reduction position is calculated based on the theoretical combustion temperature of gas, the slag-iron parameter, and gas composition after direct reduction, and details thereof are not repeated herein.

Interpolation calculation is performed on the bosh gas composition and the gas composition after direct reduction to obtain gas composition of a dripping zone, wherein the gas composition of the dripping zone includes gas compositions at different height positions of the dripping zone. Similarly, according to the above method, gas-liquid heat exchange coefficients at different height positions of the dripping zone are calculated based on the theoretical combustion temperature of gas, the slag-iron parameter, and the gas composition at each height position of the dripping zone, and details thereof are not repeated herein. By determining the gas-liquid heat exchange coefficients at different height positions of the dripping zone, accuracy of molten iron temperature prediction is further improved.

Step S150: performing iterative solution on molten iron temperature and gas temperature based on the gas-liquid heat exchange coefficient, the direct reduction degree of iron ore, the theoretical combustion temperature of gas, and the slag-iron parameter until a first convergence condition is satisfied, so as to complete prediction of molten iron temperature in blast furnace smelting.

In one embodiment of the present application, a preset temperature value may be set for molten iron temperature at a direct reduction position as a preset molten iron temperature after direct reduction. Based on the gas-liquid heat exchange coefficient, the direct reduction degree of iron ore, the theoretical combustion temperature of gas, the preset molten iron temperature after direct reduction, and the slag-iron parameter, and by using a gas-phase energy conservation relationship and a molten-iron-phase energy conservation relationship, iterative solution is performed on molten iron temperature and gas temperature until the first convergence condition is satisfied, so as to obtain gas temperature at the direct reduction position and molten iron temperature at the tuyere raceway.

Illustratively, the first convergence condition may be that an iteration number of iterative solution on molten iron temperature and gas temperature reaches a first preset iteration number, or that both a gas temperature iteration difference and a molten iron temperature iteration difference are less than or equal to a first preset temperature difference threshold, or other convergence conditions, without limitation herein.

In one embodiment of the present application, Step S150 includes: determining direct reduction heat consumption according to molten iron composition and the direct reduction degree of iron ore, wherein the slag-iron parameter includes the molten iron composition; performing iterative solution on molten iron temperature and gas temperature based on gas-phase temperature, molten-iron-phase temperature, the gas-liquid heat exchange coefficient, and the direct reduction heat consumption, and according to the gas-phase energy conservation relationship and the molten-iron-phase energy conservation relationship, to obtain a pre-iteration molten iron temperature at the tuyere raceway, a post-iteration molten iron temperature at the tuyere raceway, a pre-iteration gas temperature after direct reduction, and a post-iteration gas temperature after direct reduction, wherein an initial value of the gas-phase temperature is the theoretical combustion temperature of gas, and an initial value of the molten-iron-phase temperature is the preset molten iron temperature after direct reduction; determining a difference between the pre-iteration molten iron temperature at the tuyere raceway and the post-iteration molten iron temperature at the tuyere raceway as a molten iron temperature iteration difference, and determining a difference between the pre-iteration gas temperature after direct reduction and the post-iteration gas temperature after direct reduction as a gas temperature iteration difference; and when both the molten iron temperature iteration difference and the gas temperature iteration difference are less than or equal to the first preset temperature difference threshold, determining that the first convergence condition is satisfied, and using the post-iteration molten iron temperature at the tuyere raceway as a final prediction result of molten iron temperature prediction.

In this embodiment, an energy conservation law has the following characteristics: heat consumption caused by direct reduction of iron ore and oxides of trace elements including Si, Mn, S, P, and Ti is incorporated into gas energy variation; the energy conservation law is related to flow velocity, heat capacity, and density of gas and slag-iron; and an energy conservation calculation range includes a region from a position corresponding to start of slag-iron melting to a surface of a hearth molten pool, wherein slag-iron melting temperature ranges from 1300 °C - 1400 °C.

Therefore, iterative solution on molten iron temperature and gas temperature may be performed by using the gas-phase energy conservation relationship and the molten-iron-phase energy conservation relationship based on gas-phase temperature, molten-iron-phase temperature, the gas-liquid heat exchange coefficient, and the direct reduction heat consumption, wherein an expression of the gas-phase energy conservation relationship is as follows:
$\frac{\partial \left({ρ}_{g}{u}_{g}{C}_{g}{T}_{g}\right)}{\partial z} = - {h}_{g - l} A \left({T}_{g}-{T}_{l}\right) + \frac{{Q}_{Rd}}{{V}_{a}}$
wherein *ρ_{g}* is gas density, *u_{g}* is gas flow velocity, *C_{g}* is gas specific heat capacity, *T_{g}* is gas-phase temperature, z is a solution grid length, h*_{g-l}* is gas-liquid heat exchange coefficient, A is specific surface area, *Tₗ* is molten-iron-phase temperature, *Q_{Rd}* is direct reduction heat consumption, and *Vₐ* is a volume of a solution region.

An expression of the molten-iron-phase energy conservation relationship is as follows:
$\frac{\partial \left({ρ}_{l}{u}_{l}{C}_{l}{T}_{l}\right)}{\partial z} = {h}_{g - l} A \left({T}_{g}-{T}_{l}\right)$
wherein ρₗ is molten iron density, uₗ is molten iron flow velocity, Cₗ is molten iron specific heat capacity, Tₗ is molten-iron-phase temperature, *T_{g}* is gas-phase temperature, z is a solution grid length, h_{*g*-*l*} is gas-liquid heat exchange coefficient, and A is specific surface area.

It should be noted that the determination methods of the gas density and the gas specific heat capacity have been described in the foregoing embodiments and are not repeated herein. The specific surface area may be determined based on the slag-iron equivalent particle size; the volume of the solution region may be preset; the gas flow velocity, slag-iron flow velocity, and slag-iron equivalent particle size are all related to the blast furnace profile and may be determined in advance based on the blast furnace profile structure; and the direct reduction heat consumption may be determined based on the direct reduction degree of iron ore and the molten iron composition in the slag-iron parameter.

Please refer to Table 2, which is a table of direct reduction chemical reactions and heat effects in a specific embodiment of the present application. As shown in Table 2, oxides of elements such as Fe, Si, Mn, P, Ti, and S are converted into corresponding reduced products of Fe, Si, Mn, P, Ti, and S through direct reduction reactions.

**Table 2**

| serial number | response equation | thermal effects(kJ/mol) |
|---|---|---|
| 1 | FeO+C=Fe+CO | ΔH_{Fe} |
| 2 | SiO₂+2C=Si+2CO | ΔHₛᵢ |
| 3 | MnO+C=Mn+CO | ΔH_{Mn} |
| 4 | P₂O₅+5C=2P+5CO | ΔH_{P} |
| 5 | TiO₂+2C=Ti+2CO | ΔH_{Ti} |
| 6 | FeS+CaO+C=Fe+CaS+CO | ΔH_{S} |

Based on this, the calculation method of the direct reduction heat consumption is as follows: $\begin{matrix}{Q}_{Rd} = Δ {H}_{Fe} ⋅ Rd ⋅ \left({m}_{\left[Fe\right]}\frac{100}{1000}-M⋅{ω}_{Fe}-M⋅{ω}_{FeS}\right) + \\ Δ {H}_{Si} ⋅ \frac{{m}_{\left[Si\right]}}{28} + Δ {H}_{Mn} ⋅ \frac{{m}_{\left[Mn\right]}}{55} + Δ {H}_{P} ⋅ \frac{{m}_{\left[P\right]}}{2 \times 31} + {ΔH}_{Ti} ⋅ \frac{{m}_{\left[Ti\right]}}{48} + Δ {H}_{S} ⋅ \frac{{m}_{\left[S\right]}}{32}\end{matrix}$
wherein *Q_{Rd}* is the direct reduction heat consumption, Δ*H_{Fe}* is a reaction heat effect of direct reduction of the Fe element, *Rd* is the direct reduction degree of iron ore, *m*_{[*Fe*]} is a mass of iron in molten iron, 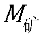 is an ore ratio, *ω_{Fe}* is a mass fraction of Fe in mixed ore,*ω_{FeS}* is a mass fraction of *FeS*(iron sulfide) in the mixed ore, Δ*H_{Si}* is a reaction heat effect of direct reduction of the Si element, *m*_{[*Si*]} is a mass of Si in molten iron, Δ*H_{Mn}* is a reaction heat effect of direct reduction of the Mn element, *m*_{[*Mn*]} is a mass of Mn in molten iron, Δ*H_{P}* is a reaction heat effect of direct reduction of the P element, *m*_{[*P*]} is a mass of P in molten iron, Δ*H_{Ti}* is a reaction heat effect of direct reduction of the Ti element, *m*_{[*Ti*]} is a mass of Ti in molten iron, Δ*H_{S}* is a reaction heat effect of direct reduction of the S element, and *m*_{[*S*]} is a mass of S in molten iron. The ore ratio, the mass fraction of Fe, and the mass fraction of FeS may be preset.

Illustratively, the dripping zone is divided into grids along a height direction. The gas-phase temperature is initialized with the theoretical combustion temperature of gas, and the molten-iron-phase temperature is initialized with a preset post-direct-reduction molten iron temperature. Iterative solutions are performed using Equation (21) and Equation (22) to obtain solution results, including a pre-iteration post-direct-reduction gas temperature, a pre-iteration molten iron temperature at the tuyere raceway, a post-iteration post-direct-reduction gas temperature, and a post-iteration molten iron temperature at the tuyere raceway.

When a difference between the pre-iteration and post-iteration post-direct-reduction gas temperatures, namely a gas temperature iterative difference, and a difference between the pre-iteration and post-iteration molten iron temperatures at the tuyere raceway, namely a molten iron temperature iterative difference, are both less than or equal to a first preset temperature difference threshold, a first convergence condition is satisfied, and the post-iteration molten iron temperature at the tuyere raceway is taken as a final prediction result of the molten iron temperature. Otherwise, iterative solutions are repeatedly performed using Equation (21) and Equation (22) until a second convergence condition is satisfied, and a final post-iteration molten iron temperature at the tuyere raceway is taken as the final prediction result of the molten iron temperature. Illustratively, the preset post-direct-reduction molten iron temperature may be 1350 °C, or any temperature within 1300-1500 °C, or another temperature value, which is not limited herein. The second preset temperature difference threshold may be 0.01 °C, or any value within 0.01-0.05 °C, or another value, which is also not limited herein.

By using the gas-phase energy conservation relationship and the molten-iron-phase energy conservation relationship, the molten iron temperature and the gas temperature are iteratively solved based on the theoretical combustion temperature of gas, the direct reduction degree of iron ore, and the gas-liquid heat exchange coefficients at different height positions, and convergence is determined based on pre- and post-iteration errors, thereby further improving accuracy of the molten iron temperature prediction.

In a specific embodiment of the present application, a 2300 m³ blast furnace is taken as an example, and the solution of the present application is further described by using a coefficient of 3.7 t/(d·m³).

A molten iron temperature prediction of a conventional blast furnace without gas injection is as follows:
Please refer to Table 3, which is a raw fuel parameter table in a specific embodiment of the present application. As shown in Table 3, the raw fuel parameters may include coke composition and coke consumption, and pulverized coal composition and consumption, wherein the coke composition may include proportions of C, H, O, N, S, volatile matter, and ash in coke, and the pulverized coal composition may include proportions of C, H, O, N, S, volatile matter, and ash in pulverized coal.

**Table 3**

| name | C | H | O | N | S | volatile matter | ash | consumption |
|---|---|---|---|---|---|---|---|---|
| unit | % | % | % | % | % | % | % | kg/t |
| coke | 86.50 | 3.43 | 2.52 | 1.70 | 1.14 | 1.07 | 12.40 | 337.69 |
| pulverized coal | 79.32 | 3.70 | 4.92 | 1.29 | 0.29 | 13.33 | 10.19 | 154.31 |

Please refer to Table 4, which is a blast parameter table in a specific embodiment of the present application. As shown in Table 4, the blast parameters may include blast volume, blast temperature, blast humidity, and oxygen enrichment ratio.

**Table 4**

| name | blast volume | blast temperature | blast humidity | oxygen enrichment ratio |
|---|---|---|---|---|
| unit | Nm3/min | °C | g/m3 | % |
| figure | 5300.20 | 1220 | 15.52 | 8.26 |

Please refer to Table 5, which is a top gas parameter table in a specific embodiment of the present application. As shown in Table 5, the top gas parameters may include top gas volume and proportions of CO, CO₂, H₂, and N₂ in the top gas.

**Table 5**

| name | H₂ | CO | CO₂ | CH₄ | N₂ | O₂ | gas injection amount |
|---|---|---|---|---|---|---|---|
| unit | % | % | % | % | % | % | m³/h |
| figure | 32.68 | 34.23 | 0.94 | 11.04 | 20.94 | 0.17 | 15000 |

Please refer to Table 6, which is a slag-iron parameter table in a specific embodiment of the present application. As shown in Table 6, the slag-iron parameters may include slag amount and proportions of Fe, C, Si, Mn, P, S, and Ti in the molten iron composition.

**Table 6**

| name | [Fe] | [C] | [Si] | [Mn] | [P] | [S] | [Ti] | slag amount |
|---|---|---|---|---|---|---|---|---|
| unit | % | % | % | % | % | % | % | kg/t |
| figure | 94.50 | 4.20 | 0.36 | 0.28 | 0.14 | 0.04 | 0.04 | 330 |

According to Table 4, the sensible heat introduced into the blast furnace by the blast, Q₁, is calculated to be 2.00 GJ/t. The oxygen amount available for combustion of pulverized coal and coke in the tuyere raceway is 239.20 m³/t, and the total heat released by combustion of pulverized coal and coke or other reactions in the tuyere raceway, Q₂, is calculated to be 2.41 GJ/t. The temperature of coke entering the combustion zone is approximately 1400 °C, and the amount of coke combusted at the tuyere is 173.46 kg/t. Accordingly, the sensible heat introduced by coke into the tuyere raceway, Q₄, is 0.29 GJ/t. Therefore, the total heat input of the tuyere raceway, Q, is 4.70 GJ/t. According to Tables 3 and 4, based on the material balance in the tuyere raceway, the contents of CO, H₂, and N₂ in the bosh gas are determined to be 41.67%, 6.95%, and 51.38%, respectively, and the bosh gas volume is 1193.88 m³/t. Accordingly, the theoretical combustion temperature of gas is calculated to be 2252 °C.

According to Tables 5 and 6, the direct reduction degree of iron ore, *Rd*, is determined to be 0.451, and after direct reduction, the gas composition after direct reduction comprises 48.92% CO, 6.09% H₂, and 44.99% N₂. According to Table 6, combined with the theoretical combustion temperature and the bosh gas volume, the Reynolds number, *Reₚ*, of the hearth is calculated to be 30883, the Prandtl number, *Pr*, is 0.362, and the Nusselt number, *Nu*, is 227.54. Accordingly, the gas-liquid heat exchange coefficient between gas and molten slag-iron droplets, *h_{g-l}*, is 26.89 W/(m²·K).

Through iterative solution based on energy conservation, the molten iron temperature, namely the molten iron temperature in the tuyere raceway, is obtained as 1521.20 °C, which is close to the average actual molten iron temperature of 1520 °C under the corresponding operating condition. The gas temperature after direct reduction is 1897.20 °C.

Since gas injection is implemented during blast furnace smelting, and the injection process causes changes in blast parameters and top gas parameters, the prediction of molten iron temperature under gas injection conditions is described as follows.

Please refer to Table 7, which is a gas injection parameter table in a specific embodiment of the present application. As shown in Table 7, the gas injection parameters may include the gas injection amount and proportions of H₂, CO, CO₂, CH₄, N₂, and O₂ in the injected gas.

**Table 7**

| name | H₂ | CO | CO₂ | CH₄ | N₂ | O₂ | gas injection amount |
|---|---|---|---|---|---|---|---|
| unit | % | % | % | % | % | % | m³/h |
| figure | 32.68 | 34.23 | 0.94 | 11.04 | 20.94 | 0.17 | 15000 |

Please refer to Table 8, which is a blast parameter table under gas injection conditions in a specific embodiment of the present application. As shown in Table 8, under the gas injection operating condition, the blast volume, blast humidity, and oxygen enrichment ratio in the blast parameters are different from the corresponding values shown in Table 4.

**Table 8**

| name | blast volume | blast temperature | blast humidity | oxygen enrichment ratio |
|---|---|---|---|---|
| unit | Nm³/min | °C | g/m³ | % |
| figure | 4857.20 | 1220 | 14.38 | 11.06 |

Please refer to Table 9, which is a top-gas parameter table under gas injection conditions in a specific embodiment of the present application. As shown in Table 9, under the gas injection operating condition, the top-gas composition and top-gas volume differ from the corresponding values shown in Table 5.

Based on Tables 3, 7, and 8, and according to the mass conservation principle in the tuyere raceway zone, the contents of CO, H₂, and N₂ in the bosh gas are obtained as 43.71%, 9.61%, and 46.68%, respectively, and the bosh gas volume is 1197.4 m³/t. Accordingly, the theoretical combustion temperature of the gas is calculated to be 2163°C. Based on Table 9, Table 6, and the bosh gas composition, the direct reduction degree of iron ore (*Rd*) is determined to be 0.39. After direct reduction, the contents of CO, H₂, and N₂ in the reduced gas are 49.90%, 8.55%, and 41.54%, respectively. Through calculation, the gas-liquid heat exchange coefficient (*h_{g-l}*) between the gas and slag-metal droplets is 28.654 W/(m²·K). Therefore, by iterative calculation based on the energy conservation law, the molten iron temperature is obtained as 1510.61°C, and the temperature of the gas after direct reduction is 1753.63°C.

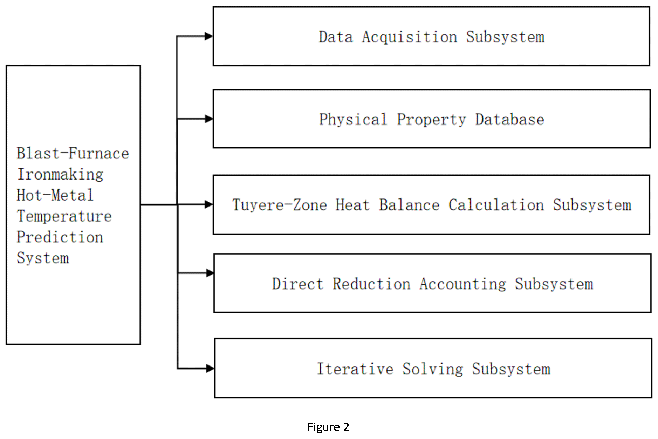

Please refer to Figure 2, which is a schematic structural diagram of a blast furnace molten iron temperature prediction system in a specific embodiment of the present application. As shown in Figure 2, the blast furnace molten iron temperature prediction system comprises a data acquisition subsystem, a physical property database, a tuyere-zone heat balance calculation subsystem, a direct reduction accounting subsystem, and an iterative solving subsystem.
The data acquisition subsystem is configured to acquire smelting data during the blast furnace operation, wherein the smelting data include raw fuel parameters, blast parameters, gas injection parameters, top-gas parameters, and slag-metal parameters.The physical property database is configured to collect and store specific heat capacity, density, dynamic viscosity, and thermal conductivity of various gases, as well as specific heat capacity, density, and thermal conductivity of various solid-phase materials. The tuyere-zone heat balance calculation subsystem is configured to calculate the thermal balance state of the tuyere zone. The direct reduction accounting subsystem is configured to calculate the direct reduction degree of iron ore. The iterative solving subsystem is configured to determine the convergence of the molten iron temperature result and to obtain the molten iron temperature value.

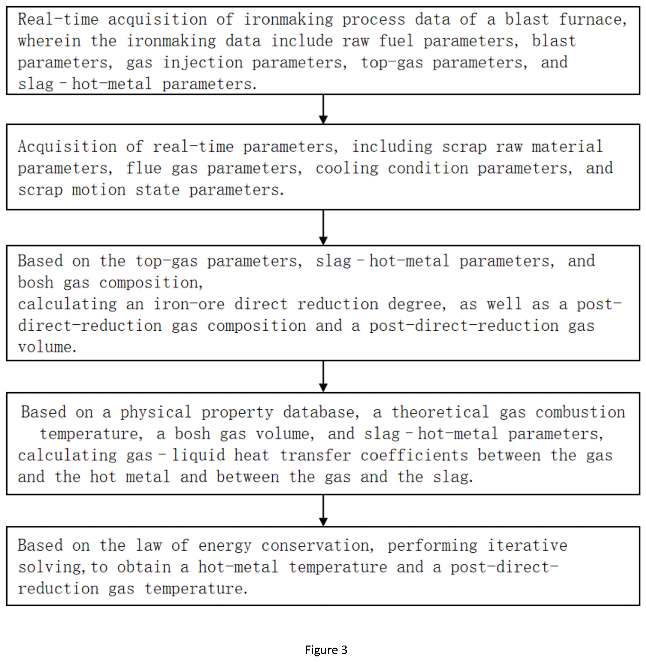

Please refer to Figure 3, which is a schematic diagram illustrating a molten iron temperature prediction process implemented by the blast furnace molten iron temperature prediction system shown in Figure 2 in a specific embodiment. As shown in Figure 3, the molten iron temperature prediction process comprises:
1.Real-time acquisition of smelting data during the blast furnace operation, wherein the smelting data include raw fuel parameters, blast parameters, gas injection parameters, top-gas parameters, and slag-metal parameters.
2.Based on the raw fuel parameters, blast parameters, and gas injection parameters, calculating the theoretical combustion temperature of the gas in the tuyere raceway, the bosh gas volume, and the bosh gas composition, respectively.
3.Based on the top-gas parameters, slag-metal parameters, and bosh gas composition, calculating the direct reduction degree of iron ore, as well as the composition and volume of the gas after direct reduction.
4.Based on the physical property database, the theoretical combustion temperature of the gas, the bosh gas volume, and the slag-metal parameters, calculating the gas-liquid heat exchange coefficients between the gas and the molten iron and between the gas and the slag.
5.Based on the energy conservation law, performing iterative solving to obtain the molten iron temperature and the temperature of the gas after direct reduction.

The detailed processes in Figure 3 may be found in the descriptions of the foregoing embodiments and are not repeated herein. The technical solution of this embodiment predicts the molten iron temperature by calculating the theoretical combustion temperature of gas in the tuyere raceway, the iron ore reduction degree, and the gas-liquid heat exchange coefficient based on real-time smelting data, and then performing iterative solving for the molten iron temperature and gas temperature. Thus, the molten iron temperature under various operating conditions can be obtained, which assists operators in evaluating the thermal state of the blast furnace, ore reduction conditions, and related information, thereby ensuring the stable operation of both conventional blast furnaces and low-carbon blast furnaces.

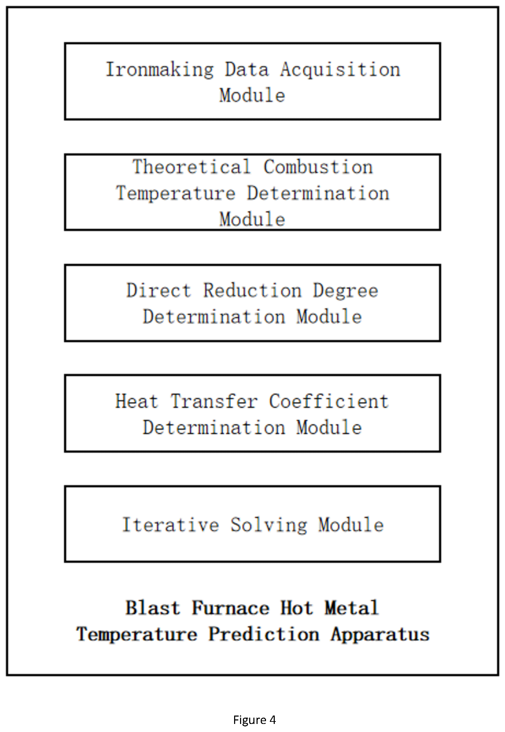

Please refer to Figure 4, which is a block diagram of a blast furnace molten iron temperature prediction apparatus according to an exemplary embodiment of the present application. As shown in Figure 4, the apparatus comprises:

a smelting data acquisition module 410, configured to acquire raw fuel parameters, blast parameters, gas injection parameters, top-gas parameters, and slag-metal parameters during the blast furnace operation; a theoretical combustion temperature determination module 420, configured to determine the total heat input, bosh gas volume, and bosh gas composition in the tuyere raceway based on the raw fuel parameters, blast parameters, and gas injection parameters, so as to determine the theoretical combustion temperature of the gas in the tuyere raceway; a direct reduction degree determination module 430, configured to determine the direct reduction degree of iron ore based on the top-gas parameters, slag-metal parameters, bosh gas volume, and bosh gas composition; a heat exchange coefficient determination module 440, configured to determine the gas-liquid heat exchange coefficient based on the theoretical combustion temperature of the gas, the bosh gas composition, the slag-metal parameters, and the composition of the gas after direct reduction, wherein the composition of the gas after direct reduction is determined based on the top-gas parameters, or based on the top-gas parameters, the bosh gas volume, and the bosh gas composition; and an iterative solving module 450, configured to perform iterative solving of the molten iron temperature and gas temperature based on the gas-liquid heat exchange coefficient, the direct reduction degree of iron ore, the theoretical combustion temperature of the gas, and the slag-metal parameters, until a first convergence condition is satisfied, thereby completing the molten iron temperature prediction.

It should be noted that the blast furnace molten iron temperature prediction apparatus described above corresponds to the blast furnace molten iron temperature prediction method described in the foregoing embodiments and is based on the same inventive concept. The specific operational details of each module have been described in detail in the method embodiments and are not repeated herein. In practical applications, the above functions may be implemented by different functional modules according to actual requirements, and the internal structure of the apparatus may be divided into different functional modules to implement all or part of the described functions, without limitation herein.

The present embodiment further provides an electronic device, comprising one or more processors and a storage device configured to store one or more programs, wherein, when the one or more programs are executed by the one or more processors, the electronic device is caused to perform the blast furnace molten iron temperature prediction method described in the foregoing embodiments.

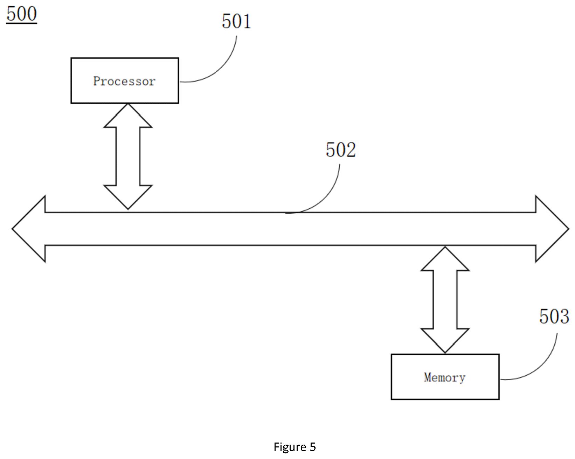

Please refer to Figure 5, which is a schematic structural diagram of an electronic device according to an exemplary embodiment of the present application. It should be noted that the electronic device 500 shown in Figure 5 is merely an example and should not impose any limitation on the functions or application scope of the embodiments of the present application.

As shown in Figure 5, the electronic device 500 comprises a processor 501, a memory 502, and a communication bus 503. The communication bus 503 is used to connect the processor 501 and the memory 502. The processor 501 is configured to execute computer programs stored in the memory 502 to implement one or more of the methods described in the foregoing embodiments.

The present embodiment further provides a computer-readable storage medium storing a computer program, wherein, when the computer program is executed by a processor of a computer, the computer performs the blast furnace molten iron temperature prediction method described above. The computer-readable storage medium may be included in the electronic device described above or may exist independently without being assembled into the electronic device.

The present embodiment further provides a computer program product or computer program, comprising computer instructions stored in a computer-readable storage medium. A processor of a computer device reads the computer instructions from the computer-readable storage medium and executes the computer instructions, thereby causing the computer device to perform the blast furnace molten iron temperature prediction method described in the foregoing embodiments.

The electronic device provided in the present embodiment comprises a processor, a memory, a transceiver, and a communication interface, wherein the memory and the communication interface are connected to the processor and the transceiver and communicate with each other. The memory is configured to store computer programs, and the communication interface is configured for communication. The processor and the transceiver are configured to run the computer programs, so that the electronic device performs the steps of the above-described methods.

In this embodiment, the memory may include a Random Access Memory (RAM) and may further include a non-volatile memory, such as at least one magnetic disk storage. The processor may be a general-purpose processor, such as a Central Processing Unit (CPU) or a Network Processor (NP), or may be a Digital Signal Processor (DSP), an Application Specific Integrated Circuit (ASIC), a Field-Programmable Gate Array (FPGA), or other programmable logic devices, discrete gate or transistor logic devices, or discrete hardware components.

A person of ordinary skill in the art will understand that all or part of the steps of the foregoing method embodiments may be implemented by hardware related to computer programs. The computer programs may be stored in a computer-readable storage medium and, when executed, perform the steps of the foregoing method embodiments. The storage medium may include ROM, RAM, magnetic disks, optical disks, or other media capable of storing program code.

The foregoing embodiments are merely illustrative of the principles and effects of the present application and are not intended to limit the present application. Any modifications or variations made by those skilled in the art without departing from the spirit and scope of the present application shall fall within the scope of protection defined by the claims of the present application.

## Claims

1. A method for predicting molten iron temperature in blast furnace smelting, the method comprising:
obtaining a raw fuel parameter, a blast parameter, a gas injection parameter, a top gas parameter, and a slag-iron parameter in a blast furnace smelting process;
determining, according to the raw fuel parameter, the blast parameter, and the gas injection parameter, total heat input, bosh gas volume, and bosh gas composition of a tuyere raceway, so as to determine theoretical combustion temperature of gas in the tuyere raceway according to the total heat input, the bosh gas volume, and the bosh gas composition;
determining a direct reduction degree of iron ore according to the top gas parameter, the slag-iron parameter, the bosh gas volume, and the bosh gas composition;
determining a gas-liquid heat exchange coefficient according to the theoretical combustion temperature of gas, the bosh gas composition, the slag-iron parameter, and gas composition after direct reduction at a direct reduction position, wherein the gas composition after direct reduction is determined based on the top gas parameter, or the gas composition after direct reduction is determined based on the top gas parameter, the bosh gas volume, and the bosh gas composition; and
performing iterative solution on the molten iron temperature and gas temperature based on the gas-liquid heat exchange coefficient, the direct reduction degree of iron ore, the theoretical combustion temperature of gas, and the slag-iron parameter until a first convergence condition is satisfied, so as to complete a prediction of the molten iron temperature in blast furnace smelting.

2. The method of claim 1, wherein the determining, according to the raw fuel parameter, the blast parameter, and the gas injection parameter, total heat input, bosh gas volume, and bosh gas composition of a tuyere raceway, so as to determine theoretical combustion temperature of gas in the tuyere raceway according to the total heat input, the bosh gas volume, and the bosh gas composition further comprising:
determining, according to the raw fuel parameter, the blast parameter, and the gas injection parameter, volume of gas participating in a chemical reaction, volume of gas not participating in the chemical reaction, and volume of gas generated by the chemical reaction in the tuyere raceway;
calculating heat released by the chemical reaction in the tuyere raceway based on the volume of gas participating in the chemical reaction, and calculating the total heat input according to coke sensible heat, blast sensible heat, gas sensible heat, and the heat released by the chemical reaction in the tuyere raceway, wherein the coke sensible heat is determined based on the raw fuel parameter, the blast sensible heat is determined based on the blast parameter, and the gas sensible heat is determined based on the gas injection parameter;
calculating the bosh gas volume and the bosh gas composition according to the volume of gas generated by the chemical reaction and the volume of gas not participating in the chemical reaction; and
performing iterative solution on the theoretical combustion temperature of gas based on the bosh gas volume, the bosh gas composition, and the total heat input until a second convergence condition is satisfied, so as to obtain the theoretical combustion temperature of gas.

3. The method of claim 2, wherein before calculating the total heat input according to the coke sensible heat, the blast sensible heat, the gas sensible heat, and the heat released by the chemical reaction in the tuyere raceway,
determining a coke specific heat capacity according to a preset coke temperature and coke composition, so as to calculate the coke sensible heat according to the preset coke temperature, the coke specific heat capacity, and a coke amount, wherein the raw fuel parameter comprises the coke composition and the coke amount;
determining a blast specific heat capacity according to a blast temperature and blast composition, so as to calculate the blast sensible heat according to the blast specific heat capacity, a blast amount, and the blast temperature, wherein the blast parameter comprises the blast composition, the blast temperature, and the blast amount; and
determining a gas injection specific heat capacity according to a preset gas injection temperature and gas injection composition, so as to calculate the gas sensible heat according to the preset gas injection temperature, the gas injection specific heat capacity, and a gas injection amount, wherein the gas injection parameter comprises the gas injection composition and the gas injection amount.

4. The method of claim 2, wherein performing iterative solution on the theoretical combustion temperature of gas based on the bosh gas volume, the bosh gas composition, and the total heat input until the second convergence condition is satisfied, so as to obtain the theoretical combustion temperature of gas, further comprising:
determining an initial bosh gas specific heat capacity of the tuyere raceway according to the bosh gas composition and a preset theoretical combustion temperature of gas, and calculating an initial theoretical combustion temperature of gas according to the total heat input, the bosh gas volume, and the initial bosh gas specific heat capacity;
using the initial theoretical combustion temperature of gas as a pre-iteration theoretical combustion temperature of gas, determining an iterative bosh gas specific heat capacity of the tuyere raceway according to the bosh gas composition and the pre-iteration theoretical combustion temperature of gas, and calculating a post-iteration theoretical combustion temperature of gas according to the total heat input, the bosh gas volume, and the iterative bosh gas specific heat capacity;
calculating a difference between the pre-iteration theoretical combustion temperature of gas and the post-iteration theoretical combustion temperature of gas to obtain an iteration temperature difference of the theoretical combustion temperature of gas;
if the iteration temperature difference of the theoretical combustion temperature of gas is less than or equal to a second preset temperature difference threshold, determining that the second convergence condition is satisfied, and using the post-iteration theoretical combustion temperature of gas as the theoretical combustion temperature of gas; and
if the iteration temperature difference of the theoretical combustion temperature of gas is greater than the second preset temperature difference threshold, performing iterative solution on the theoretical combustion temperature of gas based on the bosh gas volume, the bosh gas composition, the total heat input, and the post-iteration theoretical combustion temperature of gas until the second convergence condition is satisfied, so as to obtain the theoretical combustion temperature of gas.

5. The method of any one of claims 1-4, wherein determining the direct reduction degree of iron ore according to the top gas parameter, the slag-iron parameter, the bosh gas volume, and the bosh gas composition further comprising:
determining an amount of CO in bosh gas based on the bosh gas volume and the bosh gas composition, and determining a total amount of CO and CO₂ in top gas based on a top gas volume and a top gas composition, so as to determine a total amount of CO generated by direct reduction according to the amount of CO in bosh gas and the total amount of CO and CO₂ in top gas, wherein the top gas parameter comprises the top gas volume and the top gas composition;
determining a mass of trace elements in molten iron and a mass of iron in molten iron based on a molten iron composition and a molten iron yield, and determining an amount of CO generated by direct reduction of trace element oxides based on the mass of trace elements in molten iron, so as to determine an amount of CO generated by direct reduction of iron ore according to the total amount of CO generated by direct reduction and the amount of CO generated by direct reduction of trace element oxides, wherein the slag-iron parameter comprises the molten iron composition and the molten iron yield; and
calculating the direct reduction degree of iron ore based on the amount of CO generated by direct reduction of iron ore, the mass of iron in molten iron, and a mass of iron in a preset hot briquetted iron block.

6. The method of any one of claims 1-4, wherein, before determining the gas-liquid heat exchange coefficient,
determining a total amount of CO and CO₂ in top gas, a total amount of H₂O and H₂ in top gas, and an amount of N₂ in top gas based on a top gas volume and a top gas composition, wherein the top gas parameter comprises the top gas volume and the top gas composition;
using the top gas volume as a gas volume after direct reduction, using the total amount of CO and CO₂ in top gas as an amount of CO in gas after direct reduction, using the total amount of H₂O and H₂ in top gas as an amount of H₂ in gas after direct reduction, and using the amount of N₂ in top gas as an amount of N₂ in gas after direct reduction; and
determining the gas composition after direct reduction based on the gas volume after direct reduction, the amount of CO in gas after direct reduction, the amount of H₂ in gas after direct reduction, and the amount of N₂ in gas after direct reduction.

7. The method of any one of claims 1-4, wherein before determining the gas-liquid heat exchange coefficient,
determining a total amount of CO and CO₂ in top gas based on a top gas volume and a top gas composition, wherein the top gas parameter comprises the top gas volume and the top gas composition;
determining an amount of H₂ in bosh gas and an amount of N₂ in bosh gas based on the bosh gas volume and the bosh gas composition;
using the top gas volume as a gas volume after direct reduction, using the total amount of CO and CO₂ in top gas as an amount of CO in gas after direct reduction, using the amount of H₂ in bosh gas as an amount of H₂ in gas after direct reduction, and using the amount of N₂ in bosh gas as an amount of N₂ in gas after direct reduction; and
determining the gas composition after direct reduction based on the gas volume after direct reduction, the amount of CO in gas after direct reduction, the amount of H₂ in gas after direct reduction, and the amount of N₂ in gas after direct reduction.

8. The method of any one of claims 1-4, wherein determining the gas-liquid heat exchange coefficient according to the theoretical combustion temperature of gas, the bosh gas composition, the slag-iron parameter, and the gas composition after direct reduction at a direct reduction position further comprising:
performing interpolation calculation on the bosh gas composition and the gas composition after direct reduction to obtain a gas composition of a dripping zone;
determining a gas-liquid heat exchange coefficient of the tuyere raceway according to a slag-iron equivalent particle size, a thermal conductivity coefficient of gas in the tuyere raceway, and a Nusselt number of the tuyere raceway, wherein the thermal conductivity coefficient of gas in the tuyere raceway and the Nusselt number of the tuyere raceway are both determined based on the bosh gas composition and the theoretical combustion temperature of gas, and the slag-iron equivalent particle size is determined based on the slag-iron parameter;
determining a gas-liquid heat exchange coefficient at the direct reduction position according to the slag-iron equivalent particle size, a thermal conductivity coefficient of gas at the direct reduction position, and a Nusselt number at the direct reduction position, wherein the thermal conductivity coefficient of gas at the direct reduction position and the Nusselt number at the direct reduction position are both determined based on the gas composition after direct reduction and the theoretical combustion temperature of gas; and
determining a gas-liquid heat exchange coefficient of the dripping zone according to the slag-iron equivalent particle size, a thermal conductivity coefficient of gas in the dripping zone, and a Nusselt number of the dripping zone, wherein the thermal conductivity coefficient of gas in the dripping zone and the Nusselt number of the dripping zone are both determined based on the gas composition of the dripping zone and the theoretical combustion temperature of gas.

9. The method of claim 8, wherein performing iterative solution on the molten iron temperature and the gas temperature based on the gas-liquid heat exchange coefficient, the direct reduction degree of iron ore, the theoretical combustion temperature of gas, and the slag-iron parameter until the first convergence condition is satisfied, so as to complete molten iron temperature prediction, further comprising:
determining direct reduction heat consumption according to a molten iron composition and the direct reduction degree of iron ore, wherein the slag-iron parameter comprises the molten iron composition;
performing iterative solution on the molten iron temperature and the gas temperature based on a gas phase temperature, a molten iron phase temperature, the gas-liquid heat exchange coefficient, and the direct reduction heat consumption, and according to a gas phase energy conservation relationship and a molten iron phase energy conservation relationship, to obtain a pre-iteration molten iron temperature of the tuyere raceway, a post-iteration molten iron temperature of the tuyere raceway, a pre-iteration gas temperature after direct reduction, and a post-iteration gas temperature after direct reduction, wherein an initial value of the gas phase temperature is the theoretical combustion temperature of gas, and an initial value of the molten iron phase temperature is a preset molten iron temperature after direct reduction;
determining a difference between the pre-iteration molten iron temperature of the tuyere raceway and the post-iteration molten iron temperature of the tuyere raceway as a molten iron temperature iteration difference, and determining a difference between the pre-iteration gas temperature after direct reduction and the post-iteration gas temperature after direct reduction as a gas temperature iteration difference; and
if the molten iron temperature iteration difference and the gas temperature iteration difference are both less than or equal to a first preset temperature difference threshold, determining that the first convergence condition is satisfied, and using the post-iteration molten iron temperature of the tuyere raceway as a final prediction result of the molten iron temperature prediction.

10. A blast furnace smelting molten iron temperature prediction apparatus, the apparatus comprising:
a smelting data acquisition module, configured to obtain a raw fuel parameter, a blast parameter, a gas injection parameter, a top gas parameter, and a slag-iron parameter in a blast furnace smelting process;
a theoretical combustion temperature determination module, configured to determine, according to the raw fuel parameter, the blast parameter, and the gas injection parameter, total heat input, bosh gas volume, and bosh gas composition of a tuyere raceway, so as to determine theoretical combustion temperature of gas in the tuyere raceway according to the total heat input, the bosh gas volume, and the bosh gas composition;
a direct reduction degree determination module, configured to determine a direct reduction degree of iron ore according to the top gas parameter, the slag-iron parameter, the bosh gas volume, and the bosh gas composition;
a heat exchange coefficient determination module, configured to determine a gas-liquid heat exchange coefficient according to the theoretical combustion temperature of gas, the bosh gas composition, the slag-iron parameter, and gas composition after direct reduction at a direct reduction position, wherein the gas composition after direct reduction is determined based on the top gas parameter, or the gas composition after direct reduction is determined based on the top gas parameter, the bosh gas volume, and the bosh gas composition; and
an iterative solution module, configured to perform iterative solution on the molten iron temperature and the gas temperature based on the gas-liquid heat exchange coefficient, the direct reduction degree of iron ore, the theoretical combustion temperature of gas, and the slag-iron parameter until the first convergence condition is satisfied, so as to complete molten iron temperature prediction in blast furnace smelting.

11. An electronic device, the electronic device comprising:
one or more processors; and
a storage device, configured to store one or more programs, wherein, when the one or more programs are executed by the one or more processors, the electronic device is caused to implement the method for predicting molten iron temperature in blast furnace smelting according to any one of claims 1-9.

12. A computer-readable storage medium, wherein a computer program is stored thereon, and when the computer program is executed by a processor of a computer, the computer is caused to perform the method for predicting molten iron temperature in blast furnace smelting according to any one of claims 1-9.
